# EUROPÄISCHE PATENTSCHRIFT

(11) **EP 3 795 196 B1**
(45) Veröffentlichungstag und Bekanntmachung des Hinweises auf die Patenterteilung: **11.05.2022**
(21) Anmeldenummer: 19198038.2
(22) Anmeldetag: 18.09.2019
(51) Int. Cl.: A61M 25/00

(54) **VORRICHTUNG ZUR TEMPORÄREN, LOKALEN APPLIKATION VON FLUIDEN**
DEVICE FOR TEMPORARY LOCAL APPLICATION OF FLUIDS
DISPOSITIF D'APPLICATION TEMPORAIRE ET LOCALE DE FLUIDES

(43) Veröffentlichungstag der Anmeldung: 24.03.2021
(73) Patentinhaber: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(56) Entgegenhaltungen:
- US-A1- 2006 155 250
- US-A1- 2014 025 039
- US-A1- 2018 369 538

## Beschreibung

Die Erfindung betrifft eine Vorrichtung zur temporären, lokalen Applikation von medizinischen Fluiden, insbesondere von pharmazeutischen Fluiden. Die Erfindung betrifft auch ein Verfahren zum Betreiben einer solchen Vorrichtung.

Gegenstand der Erfindung ist insbesondere eine medizinische Vorrichtung zur temporären, lokalen Applikation von pharmazeutischen Fluiden oder anderen medizinischen Fluiden über einen Zeitraum von Stunden bis zu mehreren Tagen. Die erfindungsgemäße Vorrichtung kann je nach der jeweiligen geometrischen Anforderung beziehungsweise je nach anatomischer Situation des Implantationsorts hinsichtlich ihrer Länge durch einfaches mechanisches Kürzen angepasst werden, ohne dass ein Funktionsverlust eintritt. Weiterhin wird eine Vorrichtung zum kontinuierlichen Austragen von medizinischen Fluiden vorgeschlagen, die mit der Vorrichtung zur lokalen Applikation von medizinischen Fluiden vorteilhaft kombiniert werden kann, so dass lokal über einen Zeitraum von Stunden bis Tagen pharmazeutische Fluide oder andere medizinische Fluide kontinuierlich lokal appliziert werden können.

Die lokale Applikation von pharmazeutischen Wirkstoffen wie Antibiotika ist schon seit Jahrzehnten bekannt und bewährt sich insbesondere bei der Behandlung beziehungsweise der Beruhigung von Infektionen des Knochengewebes. Dabei kann man in nicht-resorbierbare und in resorbierbare beziehungsweise biodegradierbare Wirkstoffträger unterscheiden. Das Einleiten von Fluiden in Hohlräume zum Zweck der Spülung und Desinfektion kann aber auch zur Desinfektion und Reinigung von medizinischen Implantaten und Geräten mit Hohlräumen nützlich sein, deren Hohlräume ansonsten nur schwer zu erreichen wären.

Zur medizinischen Behandlung von Infektionen in schwer erreichbaren Hohlräumen und Kavitäten, wie Knochenhöhlen, sind resorbierbare und nichtresorbierbare Wirkstoffträger bekannt.

Exemplarisch für nichtresorbierbare Wirkstoffträger sind die seit 1977 unter dem Markennamen Septopal^{®} bekannten Kugelketten. Diese bestehen aus Polymethylmethacrylat-Kugeln, die das Breitbandantibiotikum Gentamicinsulfat enthalten, wobei diese Kugeln auf Stahlzwirn kettenförmig angeordnet sind (K. Klemm: Gentamcin-PMMA-beads in treating bone and soft tissue infections. Zentralbl. Chir. 104(14) (1979) 934-942.; K. Klemm: Antibiotic bead chains. Clin. Orthop. 295 (1993) 63-76.). Dieser kettenförmige Wirkstoffträger (Septopal^{®}) hat sich seit Jahrzehnten bei der lokalen antibiotischen Behandlung der Osteomyelitis bewährt. Vorteilhaft ist dabei, dass das Gentamicinsulfat in größeren Mengen über einen Zeitraum von mehreren Tagen aus dem Wirkstoffträger freigesetzt wird. Vorteilhaft ist es weiterhin, dass der kettenförmige Wirkstoffträger vom medizinischen Anwender problemlos durch einfaches Abschneiden des Stahlzwirns mit überzähligen Kugeln an die anatomische Situation am Implantationsort angepasst werden kann. Nachteilig ist, dass der Wirkstoffträger ausschließlich Gentamicinsulfat enthält und dass der medizinische Anwender den Wirkstoffträger nicht mit weiteren Antibiotika, entsprechend der Empfindlichkeit der mikrobiellen Keime, modifizieren kann. Zudem kann die Abgabe des pharmazeutischen Wirkstoffs nicht mehr ohne Austausch der Kugelkette an den Verlauf der Behandlung angepasst werden, wenn die Kugelkette erst einmal implantiert ist. Dadurch ist insbesondere die erfolgreiche lokale Behandlung von Infektionen mit Problemkeimen, wie MRSA und VRSA, nur bedingt oder nicht möglich. Die Entfernung der Kugelketten nach erfolgter Wirkstofffreisetzung ist durch Verwachsung mit Bindegewebe für den Patienten mit einer erheblichen Belastung verbunden.

Exemplarisch für resorbierbare beziehungsweise biodegradierbare Wirkstoffträger sind Vliese und Schwämme aus Kollagen oder Gelatine. Exemplarisch seien dafür die Druckschriften DE 34 29 038 A1, DE 33 34 595 A1, DE 28 43 963 C2, DE 32 03 957 C2 und DE 33 34 595 A1 genannt. Diese enthalten Gentamicinsulfat oder Gemische aus Gentamicinsulfat und einem in Wasser gering löslichen Gentamicinsalz. Weiterhin gibt es eine Vielzahl von resorbierbaren beziehungsweise biodegradierbaren Wirkstoffträgern auf Basis von Tricalciumphosphat, Hydroxylapatit, Gips und deren Mischungen sowie auch Kompositmaterialien aus diesen Salzen und organischen Bindemitteln. Eine Übersicht wurde von Kühn et al. publiziert (K.-D. Kühn, N. Renz, A. Trampuz: Lokale Antibiotika-Therapie. Der Unfallchirurg. 120 (2017) 561-572).

Nachteilig an den aufgeführten nicht-resorbierbaren und auch den resorbierbaren beziehungsweise biodegradierbaren Wirkstoffträgern ist, dass der antimikrobielle Wirkstoff durch die gewählte Zusammensetzung festgelegt ist und dass nach der Implantation des Wirkstoffträgers der Wirkstoff nicht mehr ausgetauscht oder mit anderen Wirkstoffen ergänzt werden kann. Weiterhin unterliegt die Wirkstofffreisetzung bei allen bisherigen lokalen Wirkstofffreisetzungssystemen auf dem Prinzip der Diffusion, so dass hohe Wirkstoffmengen nur in den ersten Stunden oder maximal ersten Tagen freigesetzt werden. Eine Ausnahme bildet die Verwendung von in Wasser gering löslichen Wirkstoffsalzen, bei denen die Wirkstofffreisetzung vom Löslichkeitsgleichgewicht der Wirkstoffsalze abhängt.

Wünschenswert ist daher ein Wirkstoffträger, der eine lokale Applikation beliebiger pharmazeutischer Wirkstoffe zulässt und wobei der pharmazeutische Wirkstoff jederzeit gegen andere fluide pharmazeutische Wirkstoffe ausgetauscht werden kann. Außerdem ist es wünschenswert, dass die Wirkstoffkonzentration, die unmittelbar am Implantationsort erreicht wird, direkt von außen eingestellt werden kann.

Aus der US 2018/0369538 A1 ist ein mehrschichtiger Katheter zum Verschließen von Blutgefäßen bekannt. Durch Aufquellen einer inneren Schicht bei Kontakt mit Flüssigkeit vergrößert sich der Katheter und so wird ein Verschluss erzeugt. Eine Applikation einer Flüssigkeit ist mit diesem Katheter nicht möglich. Die US 2006/0155250 A1 offenbart einen Verschluss zum Verschließen eines offenen Endes eines Katheters zum Applizieren einer Flüssigkeit. Mit dem Verschluss kann eine Öffnung zum Einspeisen einer Flüssigkeit in den Katheter verschlossen werden, wenn der Katheter nicht genutzt wird. Die EP 1 932 560 B1 offenbart einen Katheter zum Applizieren einer medizinischen Flüssigkeit. Der Katheter weist einen Schlauch auf, der an seinem distalen Schlauchende eine Vielzahl von Öffnungen aufweist, durch die eine Flüssigkeit aus dem Inneren des Schlauchs appliziert werden kann. Weitere ähnliche Katheter sind aus der US 5 800 407 A1, der US 6 537 194 A1, der US 2014/0025039 A1 und der US 5 425 723 A1 bekannt. Diese Katheter haben den Nachteil, dass sie eine festgelegte Länge haben, über die sie die medizinische Flüssigkeit abgeben können, und sind daher nur für bestimmte Anwendungen und für Behandlungssituationen mit bestimmten geometrischen Abmessungen einsetzbar. Die Katheter weisen also nur eine sehr geringe Variabilität zur Anpassung an die Behandlungssituation auf. Zudem ist die Abgabe der medizinischen Flüssigkeit nur über einen sich langsam abbauenden Druck einstellbar, wobei der Druck von der Elastizität der die Flüssigkeit beinhaltenden Wandungen des Katheters abhängt. Eine schnelle und kurzfristige Abgabe der medizinischen Flüssigkeit ist nicht möglich. Ferner kann bei längerem Einsatz des Katheters (über mehr als einen Tag) das den Katheter umgebende Gewebe in die Öffnungen einwachsen und so beim Herausziehen / Entfernen des Katheters erhebliche Probleme verursachen. Das umgebende Gewebe kann so durch das Entfernen des Katheters beschädigt werden und dadurch der Behandlungserfolg gemindert werden.

Der Erfindung liegt die Aufgabe zugrunde, die Nachteile des Stands der Technik zu überwinden. Insbesondere soll eine Vorrichtung zur lokalen Applikation von medizinischen Fluiden, insbesondere von pharmazeutischen Fluiden, wie zum Beispiel von antibiotischen Lösungen, bereitgestellt werden, mit der eine lokale und temporäre Abgabe des medizinischen Fluids in schwer zugänglichen Bereichen ermöglicht wird, wie zum Beispiel in Hohlräumen von nicht implantierten Implantaten oder anderen medizinischen Vorrichtungen. Die Vorrichtung soll dabei flexibel an unterschiedliche Anwendungsbereiche anzupassen sein. Eine mechanische Belastung der Wandungen der zu spülenden Hohlräume soll dabei möglichst vermieden werden. Bei der Verwendung zur Behandlung einer Infektion soll eine möglichst schonende Behandlung möglich sein, bei der das angrenzende entzündete Gewebe möglichst wenig gereizt wird und zwar sowohl bei einer temporären Abgabe des Fluids als auch beim Einsetzen und Entfernen des eingeführten Teils der Vorrichtung. Die Vorrichtung soll auch über längere Zeiträume an einem bestimmten Ort zur wiederholten Abgabe des Fluids geeignet sein, ohne dass die Vorrichtung hierfür entfernt werden muss. Die Vorrichtung soll kostengünstig zu fertigen sein und möglichst ein hygienisches, nur einmal verwendbares Wegwerfprodukt sein. Dabei soll zumindest der in den zu spülenden Hohlraum platzierbare Teil der Vorrichtung oder eben die gesamte Vorrichtung als Wegwerfprodukt kostengünstig und leicht entsorgbar sein.

Die Aufgabe der Erfindung besteht somit auch darin, eine einfache, kostengünstige Vorrichtung zur lokalen Applikation von medizinischen Fluiden zu entwickeln. Die Vorrichtung soll eine lokale Applikation insbesondere von pharmazeutischen Fluiden mit beliebiger Zusammensetzung, zum Beispiel antibiotischen Lösungen, ermöglichen. Ein Teil der Vorrichtung befindet sich bei einer medizinischen Anwendung nach einer Implantation im Patienten und ein zweiter Teil der Vorrichtung außerhalb des Patienten. Die medizinischen Fluide sollen in dem außerhalb des im Patienten befindlichen Teils der Vorrichtung eingebracht werden können und zum Implantationsort durch die Vorrichtung geleitet und dort freigesetzt werden können. Die Vorrichtung soll plastisch verformbar sein, um den anatomischen Gegebenheiten am Implantationsort oder der geometrischen Form der Hohlform folgen zu können. Nach der erfolgten Formgebung durch den medizinischen Anwender soll die Gestalt der Vorrichtung sich nicht verändern können außer durch manuelle Verformung durch den medizinischen Anwender.

Die Freisetzung von pharmazeutischen Fluiden soll aus längs an der Vorrichtung angeordneten Öffnungen erfolgen. Die Öffnungen sollen reversibel verschließbar sein, um einen Rückfluss von kontaminiertem Fluid in das Innere der Vorrichtung zu verhindern beziehungsweise um ein Einwachsen von Bindegewebe beziehungsweise eine Verstopfung der Öffnungen durch koaguliertes Blut zu verhindern. Weiterhin soll die Vorrichtung so beschaffen sein, dass der gegebenenfalls im Patienten befindliche Teil der Vorrichtung durch Kürzen der Länge an die jeweilige anatomische Situation des Patienten angepasst werden kann, ohne dass die Funktion der Vorrichtung beeinträchtigt wird.

Die Vorrichtung darf sich außerdem nicht bei der Applikation des medizinischen Fluids beziehungsweise des pharmazeutischen Fluids hinsichtlich der Gestalt und auch hinsichtlich des Durchmessers signifikant verändern. Durch eine signifikante Querdehnung könnte es ansonsten zu Schmerzen am entzündeten beziehungsweise infizierten Gewebe beim Patienten kommen. Weiterhin soll eine einfache kostengünstige Vorrichtung entwickelt werden, die einen kontinuierlichen Austrag von medizinischen Fluiden, insbesondere von pharmazeutischen Fluiden, über einen Zeitraum von Stunden bis Tagen ermöglicht, ohne dass Elektromotoren, Batterien oder Akkumulatoren erforderlich sind.

Die Aufgaben der Erfindung werden gelöst durch eine Vorrichtung zur lokalen Applikation eines medizinischen Fluids aufweisend einen Schlauch, der flexibel verformbar ist und der eine Schlauchwand aufweist, wobei die Schlauchwand eine äußere Wandung aus einem ersten Material aufweist, die radial außen liegend angeordnet ist, und die Schlauchwand eine innere Wandung aus einem zweiten Material aufweist, die radial innen liegend angeordnet ist und die eine innere Leitung des Schlauchs begrenzt, wobei der Schlauch in einem distalen Teilstück des Schlauchs mehrere Öffnungen in der Schlauchwand aufweist, wobei die mehreren Öffnungen die innere Leitung des Schlauchs mit der Umgebung des Schlauchs verbinden, wobei das distale Teilstück des Schlauchs von einem distalen Ende des Schlauchs begrenzt ist, wobei die mehreren Öffnungen in der inneren Wandung abhängig von einer auf das zweite Material wirkenden physikalischen Größe, insbesondere abhängig von einem von dem medizinischen Fluid auf das zweite Material wirkenden Druck, reversibel fluiddicht verschließbar sind oder die mehreren Öffnungen in der äußeren Wandung abhängig von einer auf das erste Material wirkenden physikalischen Größe, insbesondere abhängig von einem von dem medizinischen Fluid auf das erste Material wirkenden Druck, reversibel fluiddicht verschließbar sind, wobei der Schlauch in einem proximalen Teilstück des Schlauchs keine Öffnungen in der Schlauchwand aufweist, die die innere Leitung des Schlauchs mit der Umgebung des Schlauchs flüssigkeitsdurchlässig verbinden, die Vorrichtung ferner aufweisend ein Verschlusselement, mit dem der Schlauch an dem distalen Ende des Schlauchs flüssigkeitsdicht verschlossen ist oder verschließbar ist, wobei das Verschlusselement manuell in das distale Ende des Schlauchs einsetzbar ist, wobei ein proximales Ende des Schlauchs derart mit einem Behälter für das medizinische Fluid flüssigkeitsdurchlässig verbunden ist oder verbindbar ist, dass das medizinische Fluid aus dem Behälter durch das proximale Ende des Schlauchs in die innere Leitung des Schlauchs drückbar ist und durch die mehreren Öffnungen an die Umgebung des Schlauchs herausdrückbar ist.

Mit der Vorrichtung können auch medizinische Instrumente und nicht implantierte Implantate abgespült oder ausgespült werden, insbesondere medizinischen Instrumente und Implantate mit Hohlräumen, in die der Schlauch eingeführt werden kann. Die Vorrichtung kann aber auch zum freien Verteilen des medizinischen Fluids verwendet werden. Besonders geeignet ist jedoch eine medizinische Anwendung der erfindungsgemäßen Vorrichtung, bei der der Schlauch in eine Kavität eines menschlichen Körpers eingeführt wird und das Fluid zur Behandlung des angrenzenden Gewebes eingesetzt wird.

Die erfindungsgemäße Vorrichtung ist bevorzugt eine medizinische Vorrichtung.

Das erste Material und das zweite Material unterscheiden sich bevorzugt hinsichtlich zumindest einer Materialeigenschaft voneinander. Besonders bevorzugt unterscheiden sich das erste Material und das zweite Material hinsichtlich der Elastizität und/oder Härte voneinander.

Gemäß einer bevorzugten Weiterbildung der vorliegenden Erfindung kann das zweite Material ein gummielastisches Material sein, während das erste Material formstabiler als das zweite Material ist. Beispielsweise kann die innere Wandung eine Beschichtung mit einem gummielastischen Material auf der Innenseite der äußeren Wandung sein.

Die Richtungsbezeichnungen distal und proximal beziehen sich vorliegend auf die in der Anwendung vorgesehene Flussrichtung des medizinischen Fluids. Das medizinische Fluid fließt dabei von einem proximalen Ende des Schlauchs in Richtung zum distalen Ende des Schlauchs und dort aus den mehreren Öffnungen.

Die Schlauchwand kann mantelförmig geformt sein.

Die äußere Wandung kann die innere Wandung mantelförmig umgeben.

Bevorzugt ist der Schlauch bis auf die mehreren Öffnungen zylindrisch geformt. Die Schlauchwand begrenzt den Schlauch dann besonders bevorzugt an seiner Zylindermantelfläche. Bei geraden Schläuchen mit einer zylindrischen Geometrie ist die Mantelfläche die Wandung senkrecht zur Zylinderachse des zylindrischen Schlauchs. Die Öffnungen befinden sich also in der Mantelfläche.

Ferner kann vorgesehen sein, dass das proximale Teilstück des Schlauchs von dem proximalen Ende des Schlauchs begrenzt ist.

Des Weiteren kann vorgesehen sein, dass der Schlauch am distalen Ende mit dem Verschlusselement gasdicht und/oder druckdicht verschlossen ist oder verschließbar ist.

Als medizinisches Fluid wird bevorzugt ein pharmazeutisches Fluid verwendet. Ein pharmazeutisches Fluid enthält wenigstens einen pharmazeutischen Wirkstoff. Besonders bevorzugt werden Lösungen enthaltend wenigstens ein Antibiotikum, wenigstens ein Zytostatikum, wenigstens ein Chemotherapeutikum und/oder wenigstens ein Antimykotikum pharmazeutische Fluide beziehungsweise als medizinische Fluide. Alternative medizinische Fluide können desinfizierende Bestandteile enthalten. Unter dem Begriff "pharmazeutisches Fluid" werden demzufolge wässrige und auch nichtwässrige Lösungen und Suspensionen von pharmazeutischen Wirkstoffen verstanden. Weiterhin werden unter dem Begriff "pharmazeutisches Fluid" auch Gemische und Lösungen von Gasen in Wasser, Wasser enthaltenden Flüssigkeiten und nichtwässrigen Flüssigkeiten verstanden. Der Begriff "pharmazeutisches Fluid" umfasst bevorzugt auch Gase und Gasgemische.

Es kann auch vorgesehen sein, dass zumindest eine der mehreren Öffnungen im Bereich des distalen Endes des Schlauchs angeordnet ist, bevorzugt innerhalb von 5 mm des distalen Endes des Schlauchs angeordnet ist, besonders bevorzugt innerhalb von 3 mm des distalen Endes des Schlauchs angeordnet ist.

Bevorzugt sind in der Schlauchwandung zumindest drei Öffnungen als die mehreren Öffnungen angeordnet.

Bevorzugt kann vorgesehen sein, dass in dem Verschlusselement ein röntgenopakes Material enthalten ist oder dass das Verschlusselement aus einem röntgenopaken Material besteht. Das röntgenopake Material kann besonders bevorzugt ausgewählt sein aus Edelstahl, Titan, Titanlegierungen, Tantal, Tantallegierungen, Bariumsulfat, Bariumsulfat enthaltende Kunststoffe, Zirkoniumdioxid und Zirkoniumdioxid enthaltende Kunststoffe.

Bei erfindungsgemäßen Vorrichtungen kann vorgesehen sein, dass die innere Leitung des Schlauchs an einer proximalen Öffnung im proximalen Ende des Schlauchs beginnt und an einer distalen Öffnung im distalen Ende des Schlauchs endet, wobei die distale Öffnung des Schlauchs durch das Verschlusselement verschlossen ist oder verschließbar ist.

Die innere Leitung kann so die beiden offenen Enden, nämlich das distale Ende und das proximale Ende des Schlauchs, verbinden. Hierdurch kann das medizinische Fluid durch die innere Leitung des Schlauchs geleitet und durch die mehreren Öffnungen in der Schlauchwand appliziert werden.

Des Weiteren kann vorgesehen sein, dass die Vorrichtung den Behälter für das medizinische Fluid aufweist, wobei bevorzugt der Behälter einen Hohlzylinder mit einem axial im Hohlzylinder verschiebbaren Kolben aufweist, der ein erstes Ende des Hohlzylinders verschließt, wobei der Hohlzylinder an einem dem ersten Ende gegenüberliegenden Ende eine Austragsöffnung aufweist, die mit dem proximalen Ende des Schlauchs verbunden oder verbindbar ist, bevorzugt über ein manuell bedienbares Ventilelement zur Regulierung der Strömungsgeschwindigkeit des medizinischen Fluids mit dem proximalen Ende des Schlauchs verbunden oder verbindbar ist.

Hierdurch muss kein separates Reservoir für die medizinische Flüssigkeit an der Vorrichtung angeschlossen werden. Bevorzugt ist der Kolben mit zumindest einer gespannten elastischen Feder antreibbar.

Dabei kann vorgesehen sein, dass in dem Behälter ein medizinisches Fluid, insbesondere ein pharmazeutisches Fluid, enthalten ist.

Hierdurch ist die Vorrichtung unmittelbar zum Erzeugen eines Stroms des medizinischen Fluids aus den mehreren Öffnungen einsetzbar.

Bevorzugt kann des Weiteren vorgesehen sein, dass die Vorrichtung eine Fördereinrichtung aufweist, mit der das medizinische Fluid aus dem verbundenen oder verbindbaren Behälter in den Schlauch, durch die innere Leitung des Schlauchs und durch die mehreren Öffnungen in die Umgebung des Schlauchs zu drücken ist.

Damit kann die Vorrichtung auch zum Antreiben des Stroms der medizinischen Flüssigkeit verwendet werden. Mit einer solchen Vorrichtung ist es möglich, über einen Zeitraum von Stunden bis zu mehreren Tagen pharmazeutische Fluide lokal zu applizieren, ohne dass aufwendige elektrisch betriebene Pumpsysteme notwendig sind. Bevorzugt kann mit der Fördereinrichtung das medizinische Fluid diskontinuierlich oder kontinuierlich gefördert werden.

Bei Vorrichtungen mit Fördereinrichtung kann bevorzugt vorgesehen sein, dass die Fördereinrichtung ein Energiespeicherelement, insbesondere zumindest eine gespannte Feder, aufweist, wobei die Fördereinrichtung mit Energie aus dem Energiespeicherelement antreibbar ist, wobei vorzugsweise mit dem Energiespeicherelement ein Kolben in einem Hohlzylinder als der Behälter in Richtung einer gegenüberliegenden Austragsöffnung anzutreiben ist.

Dadurch muss die Vorrichtung nicht an eine externe Energieversorgung zum Antreiben der Fördereinrichtung angeschlossen werden. Eine gespannte Feder enthält dabei genug Energie, um eine Menge von einigen Millilitern bis einigen Zentilitern des medizinischen Fluids mit der Vorrichtung auszupressen.

Es kann auch vorgesehen sein, dass die äußere Wandung und die innere Wandung miteinander fest verbunden sind, bevorzugt vollflächig miteinander verbunden sind.

Hiermit sind die äußere Wandung und die innere Wandung gegeneinander fixiert. Dadurch ist es möglich, dass die mehreren Öffnungen in der inneren Wandung sich aufgrund einer elastischen Entspannung ohne einen Druck des medizinischen Fluids in der inneren Leitung verschließen können, während die äußere Wandung den Druck aufnehmen kann, der zum Öffnen der mehreren Öffnungen in der inneren Wandung notwendig ist.

Zur wirksamen Fluidabgabe kann vorgesehen sein, dass die Summe der freien Querschnittsflächen aller der mehreren Öffnungen maximal so groß ist wie der freie Querschnitt der inneren Leitung.

Hierdurch wird sichergestellt, dass auch die Öffnungen der mehreren Öffnungen, die am distalen Ende des Schlauchs angeordnet sind, von dem medizinischen Fluid durchströmt werden können. Damit wird sichergestellt, dass auch aus den am distalen Ende angeordneten Öffnungen noch medizinisches Fluid austritt. Die Summe der freien Querschnittsflächen aller der mehreren Öffnungen bezieht sich den geöffneten Zustand der mehreren Öffnungen.

Ferner kann vorgesehen sein, dass die mehreren Öffnungen in der äußeren Wandung der Schlauchwand unabhängig vom Druck des medizinischen Fluids offen sind, während die mehreren Öffnungen in der inneren Wandung der Schlauchwand ohne Druckbeaufschlagung durch das medizinische Fluid verschlossen sind und durch einen Druck auf das medizinische Fluid flüssigkeitsdurchlässig zu öffnen sind.

Hierdurch verschließen sich die mehreren Öffnungen in der Schlauchwand, wenn keine medizinische Flüssigkeit in die innere Leitung gedrückt wird. Bei einem alternierenden Betrieb kann so verhindert werden, dass Gewebe durch die mehreren Öffnungen in die innere Leitung wächst und so die Vorrichtung in der Kavität verwächst.

Bevorzugt kann auch vorgesehen sein, dass die äußere Wandung der Schlauchwand einen über die innere Wandung der Schlauchwand vermittelten Druck des medizinischen Fluids in der inneren Leitung aufnimmt, ohne sich dabei radial um mehr als 5% auszudehnen, bevorzugt ohne sich dabei radial um mehr als 1% auszudehnen.

Der Druck der medizinischen Flüssigkeit kann sich dabei unter normalen Bedingungen bei normalen Anwendungen der erfindungsgemäßen Vorrichtung nicht über 500 kPa erhöhen. Es kann also vorgesehen sein, dass die äußere Wandung der Schlauchwand einen über die innere Wandung der Schlauchwand vermittelten hydrostatischen Druck von maximal 500 kPa in der inneren Leitung aufnimmt, ohne sich dabei radial um mehr als 5% auszudehnen, bevorzugt ohne sich dabei radial um mehr als 1% auszudehnen.

Hierdurch wird sichergestellt, dass sich der Schlauch nicht zu stark ausdehnt, wenn das medizinische Fluid durch den Schlauch gedrückt wird. Hiermit wird eine Irritation des umgebenden Gewebes verhindert oder eine mechanische Belastung der umgebenden Strukturen verhindert.

Mit der vorliegenden Erfindung wird auch vorgeschlagen, dass sich der Schlauch bei einem Innendruck von 500 kPa gegenüber Normaldruck sich radial um maximal 5 Prozent ausdehnt, bevorzugt um maximal 1 Prozent ausdehnt.

Hierdurch wird sichergestellt, dass sich der Schlauch nicht zu stark ausdehnt, wenn das medizinische Fluid durch den Schlauch gedrückt wird. Hiermit wird eine Irritation des umgebenden Gewebes verhindert oder eine mechanische Belastung der umgebenden Strukturen verhindert.

Des Weiteren kann vorgesehen sein, dass das Verschlusselement einen konischen oder zylindrischen Vorsprung aufweist, der in den Schlauch gesteckt oder geschraubt ist, so dass der Schlauch im Bereich des distalen Endes des äußeren Schlauchs durch den Vorsprung derart gespannt ist, dass der Schlauch am distalen Ende des Schlauchs fluiddicht verschlossen ist, wobei vorzugsweise der konische oder zylindrische Vorsprung Stege an der Außenseite des Vorsprungs aufweist oder der konische oder zylindrische Vorsprung ein Außengewinde aufweist, wobei das Außengewinde oder der konische oder zylindrische Vorsprung einen größeren Außendurchmesser aufweist als der Innendurchmesser des Schlauchs.

Hiermit lässt sich das distale Ende des Schlauchs auch nach einem Kürzen des distalen Teilstücks des Schlauchs zuverlässig abdichten.

Gemäß einer bevorzugten Weiterbildung der vorliegenden Erfindung kann vorgesehen sein, dass die mehreren Öffnungen im distalen Teilstück des Schlauchs in der äußeren Wandung einen Durchmesser von maximal 500 µm, bevorzugt von maximal 250 µm und besonders bevorzugt von maximal 100 µm haben.

Der Durchmesser bezieht sich dabei auf den mittleren Durchmesser des Öffnungsquerschnitts der mehreren Öffnungen. Wenn die innere Wandung dazu ausgelegt ist, die mehreren Öffnungen im entspannten Zustand, also bei fehlender Druckbeaufschlagung, zu verschließen, ist der Durchmesser der inneren Wandung zumindest im geschlossenen Zustand natürlich kleiner. Im geöffneten Zustand der mehreren Öffnungen in der inneren Wandung ist der Durchmesser der mehreren Öffnungen in der inneren Wandung maximal so groß wie der Durchmesser der mehreren Öffnungen in der äußeren Wandung.

Bei Öffnungen mit solchen maximalen Durchmessern ist sichergestellt, dass die Durchflussraten des medizinischen Fluids nicht zu hoch werden und andererseits der freie Leitungsquerschnitt der inneren Leitung dazu ausreicht, auch die Öffnungen nahe dem distalen Ende des Schlauchs nur zur Abgabe des medizinischen Fluids nutzen zu können.

Bevorzugte Ausführungsformen der vorliegenden Erfindung können sich auch dadurch auszeichnen, dass der Schlauch aus einem koaxialen Koextrudat gebildet ist, wobei die innere Wandung aus einem gummielastischen Polymer, insbesondere Polyurethan oder einem schwach vernetzten Polymer, besteht und die äußere Wandung aus einem nicht gummielastischen thermoplastischen Polymer oder aus einem stark vernetzten Polymer besteht, insbesondere aus Polyamid besteht.

Hierdurch wird erreicht, dass die Öffnungen durch die innere Wandung verschlossen sind, wenn kein hydrostatischer Druck auf die innere Wandung ausgeübt wird, und sich die Öffnungen in der inneren Wandung öffnen, wenn der von dem medizinischen Fluid ausgeübte Druck ansteigt. Dadurch kann ein Einwachsen von Gewebe und eine Verunreinigung der inneren Leitung durch die Öffnungen verhindert werden.

Besonders bevorzugt sind koextrudierte Schläuche bei denen die inneren Wandung aus einem gummielastischen Polyurethan besteht und die äußere Wandung aus thermoplastischem, nicht gummielastischem Polyamid gebildet ist. Die mehreren Öffnungen gehen durch die innere Wandung und die äußere Wandung. Bei Druckbeaufschlagung mit einem medizinischen Fluid werden die Öffnungen in der inneren Wandung durch elastisches Zurückweichen des elastischen Polyurethans freigeben und das medizinische Fluid kann durch die Öffnungen des starren Polyamids aus der äußeren Wandung austreten. Nach Beendigung des Fluidaustrags verschließen sich die Öffnungen in der inneren Wandung des Schlauchs wieder und Körperflüssigkeiten, wie zum Beispiel Blut oder Wundexsudat, können nicht in die innere Leitung des Schlauchs eindringen. Währen des Fluidaustrags verhindert die starre, nichtelastische äußere Wandung eine radiale Dehnung des Schlauchs. Dadurch werden keine Kräfte auf das zu behandelnde Gewebe ausgeübt und Schmerzen infolge einer mechanischen Einwirkung verhindert, beziehungsweise keine Kräfte auf die Wandungen des gespülten Implantats oder Hohlraums übertragen.

Es kann auch vorgesehen sein, dass zumindest am distalen Ende des Schlauchs und/oder im Verschlusselement ein röntgenopakes Material im Schlauch enthalten ist, bevorzugt im distalen Teilstück des Schlauchs und/oder im Verschlusselement ein röntgenopakes Material enthalten ist, besonders bevorzugt auf der gesamten Länge des distalen Teilstücks des Schlauchs und im Verschlusselement enthalten ist oder auf der gesamten Länge des Schlauchs und im Verschlusselement enthalten ist.

Hierdurch kann die Position und Lage des Schlauchs durch Röntgenverfahren sichtbar gemacht werden und der Schlauch unter Röntgenkontrolle positioniert werden und dadurch die Lage der Vorrichtung im Patienten durch Röntgenaufnahmen eindeutig bestimmt werden.

Das röntgenopake Material kann besonders bevorzugt ausgewählt sein aus Edelstahl, Titan, Titanlegierungen, Tantal, Tantallegierungen, Bariumsulfat, Bariumsulfat enthaltende Kunststoffe, Zirkoniumdioxid und Zirkoniumdioxid enthaltende Kunststoffe.

Ferner kann vorgesehen sein, dass zumindest ein Metalldraht, zumindest eine Metallspirale und/oder zumindest ein Metallnetz in der inneren Leitung des Schlauchs und/oder in der Schlauchwand des Schlauchs angeordnet ist oder sind, wobei der zumindest eine Metalldraht, die zumindest eine Metallspirale und/oder das zumindest ein Metallnetz bevorzugt entlang der gesamten Länge des Schlauchs angeordnet sind.

Der zumindest eine Metalldraht, die zumindest eine Metallspirale und das zumindest eine Metallnetz dienen der plastischen Verformbarkeit des Schlauchs. Dadurch kann die Form des Schlauchs verändert und damit an die jeweilige Situation angepasst werden, ohne dass die Umgebung der Vorrichtung mechanisch beansprucht wird, wobei die Form des Schlauchs von dem zumindest einen Metalldraht, der zumindest einen Metallspirale und/oder dem zumindest ein Metallnetz gehalten wird. So behält die Vorrichtung nach vorheriger Formung entsprechend den anatomischen Gegebenheiten ihre Form bei. Dadurch ist ein ortsgenaues Applizieren von pharmazeutischen Fluiden an genau vorherbestimmten Implantationsorten möglich. Zudem sind die metallischen Strukturen im Röntgenbild erkennbar.

Bevorzugt kann auch vorgesehen sein, dass das Verschlusselement die folgenden Merkmale aufweist: einen rotationssymmetrischen ersten Körper mit Außengewinde oder mit am Umfang umlaufenden Stegen, wobei das Außengewinde oder die Stege einen größeren Außendurchmesser besitzt als der Innendurchmesser des Schlauchs, einen rotationssymmetrischen zweiten Körper mit einem Außendurchmesser kleiner oder gleich dem Außendurchmesser des Schlauchs, wobei die axiale Erstreckung des zweiten Körpers mindestens 5 mm ist, wobei der rotationssymmetrische erste Körper axial mit dem rotationssymmetrischen zweiten Körper verbunden ist.

Hierdurch kann der Schlauch mit dem Verschlusselement zuverlässig und flüssigkeitsdicht verschlossen werden.

Des Weiteren kann vorgesehen sein, dass das Verschlusselement in das distale Ende des Schlauchs eingeschraubt oder eingepresst ist und den freien Leitungsquerschnitt der inneren Leitung des Schlauchs am distalen Ende vollständig flüssigkeitsdicht und druckdicht verschließt.

Hierdurch wird sichergestellt, dass in der inneren Leitung ein ausreichender Druck mit dem medizinischen Fluid aufgebaut werden kann, um die medizinische Flüssigkeit aus allen der mehreren Öffnungen drücken zu können. Bevorzugt verschließt das Verschlusselement das distale Ende des Schlauchs auch gasdicht.

Es kann ferner vorgesehen sein, dass am proximalen Ende des Schlauchs oder in der Verbindung zu dem Behälter für das medizinische Fluid ein Ventilelement, insbesondere ein Rückschlagventil, angeordnet ist, das eine Strömung des medizinischen Fluids in Richtung des Behälters verhindert und eine Strömung des medizinischen Fluids aus dem Behälter in Richtung des distalen Teilstücks verhindert.

Hierdurch wird sichergestellt, dass kein kontaminiertes medizinisches Fluid aus der inneren Leitung in den Behälter für das medizinische Fluid vordringen kann.

Auch kann vorgesehen sein, dass die mehreren Öffnungen alle, paarweise oder gruppenweise in axialer Richtung des äußeren Schlauchs zueinander beabstandet sind.

Hierdurch kann das medizinische Fluid an verschiedenen axial voneinander beabstandeten Stellen austreten. Zudem kann das distale Teilstück des Schlauchs an verschiedenen Stellen in der Länge gekürzt werden, wobei gleichzeitig immer noch wenigstens eine Öffnung der mehreren Öffnungen in dem distalen Teilstück des Schlauchs vorhanden ist.

Bevorzugt kann auch vorgesehen sein, dass der Schlauch plastisch verformbar ist und/oder einen Außendurchmesser kleiner oder gleich 7 mm aufweist, bevorzugt einen Außendurchmesser zwischen 2 mm und 4 mm.

Durch den geringen Außendurchmesser ist der Schlauch gut in Kavitäten von Implantaten und in Kavitäten im menschlichen Körper einführbar und dort zum Ausspülen der Kavitäten geeignet. Durch die plastische Verformbarkeit wird verhindert, dass eine elastische Kraft auf die Wandungen des zu spülenden Hohlraums wirkt und diesen mechanisch belastet.

Des Weiteren kann vorgesehen sein, dass das erste Material eine größere Shore A-Härte hat als das zweite Material, wobei bevorzugt das erste Material eine Shore A-Härte von mehr als 60 und das zweite Material eine Shore A-Härte von weniger als 60 hat.

Die Shore-Härte wird dazu nach DIN ISO 7619-1 (2012-02) [2] bestimmt. Durch Materialien mit diesen Härteunterschieden wird sichergestellt, dass die innere Wandung die mehreren Öffnungen in der äußeren Wandung verschließen kann.

Ferner kann vorgesehen sein, dass die mehreren Öffnungen in der inneren Wandung bei Druckbeaufschlagung mit einem hydrostatischen Druck von 500 kPa durch das medizinische Fluid einen um den Faktor zwei oder mehr größeren freien Querschnitt aufweisen als ohne Druckbeaufschlagung.

Hiermit wird sichergestellt, dass die mehreren Öffnungen in der inneren Wandung durch den Druck des medizinischen Fluids zu öffnen sind.

Es kann vorgesehen sein, dass die mehreren Öffnungen in der inneren Wandung oder der äußeren Wandung schlitzförmig geformt sind.

Durch diese beiden Maßnahmen wird erreicht, dass sich die schlitzförmigen Öffnungen durch einen auf das medizinische Fluid wirkenden Druck öffnen lassen und sich bei Reduzieren des Drucks auf das medizinische Fluid wieder verschließen. Dadurch kann ein Einwachsen von Gewebe in die mehreren Öffnungen bei alternierendem Betrieb vermieden werden. Auch kann so eine Verunreinigung des medizinischen Fluids in der inneren Leitung verhindert werden. Bevorzugt sind die mehreren Öffnungen in der inneren Wandung schlitzförmig geformt. Das hat den Vorteil, dass deren Verformung keine Verformung der Außenflächen des Schlauchs verursacht.

Es kann vorgesehen sein, dass die mehreren Öffnungen in der inneren Wandung abhängig von einer auf das zweite Material wirkenden physikalischen Größe, insbesondere abhängig von einem von dem medizinischen Fluid auf das zweite Material wirkenden Druck, reversibel fluiddicht verschließbar sind oder die mehreren Öffnungen in der äußeren Wandung abhängig von einer auf das erste Material wirkenden physikalischen Größe, insbesondere abhängig von einem von dem medizinischen Fluid auf das erste Material wirkenden Druck, reversibel fluiddicht verschließbar sind.

Im zweiten Fall kann der Druck in den offenen Teilen der mehreren Öffnungen in der inneren Wandung auf das erste Material der äußeren Wandung wirken. Bevorzugt sind das erste Material und das zweite Material derart gewählt, dass die mehreren Öffnungen in der inneren Wandung abhängig von einer auf das zweite Material wirkenden physikalischen Größe, insbesondere abhängig von einem von dem medizinischen Fluid auf das zweite Material wirkenden Druck, reversibel fluiddicht verschließbar sind oder die mehreren Öffnungen in der äußeren Wandung abhängig von einer auf das erste Material wirkenden physikalischen Größe, insbesondere abhängig von einem von dem medizinischen Fluid auf das erste Material wirkenden Druck, reversibel fluiddicht verschließbar sind.

Durch diese Maßnahmen können die mehreren Öffnungen reversibel geöffnet und verschlossen werden. Neben dem Druck kann beispielsweise eine elektrische oder mechanische Spannung, ein Magnetfeld oder eine Temperatur (beispielsweise durch den Einsatz von Shape-Memory-Alloys) zum Öffnen und Schließen der mehreren Öffnungen verwendet werden.

Ferner kann vorgesehen sein, dass die mehreren Öffnungen durch eine elastische Deformation des zweiten Materials reversibel zu öffnen sind, während die mehreren Öffnungen im ersten Material offen bleiben, wobei vorzugsweise das erste Material derart formfest ist, dass die äußere Wandung zumindest einen Teil der durch die elastische Verformung des zweiten Materials verursachten Kräfte aufnimmt und so einer radialen Verformung des Schlauchs entgegenwirkt.

Hierdurch wird erreicht, dass der Schlauch sich nicht oder nur sehr gering radial verformt.

Alternativ kann auch vorgesehen sein, dass die mehreren Öffnungen durch eine elastische Deformation des ersten Materials reversibel zu öffnen sind, während die mehreren Öffnungen im zweiten Material offen bleiben, wobei vorzugsweise das zweite Material derart formfest ist, dass die innere Wandung zumindest einen Teil der durch die elastische Verformung des ersten Materials verursachten Kräfte aufnimmt und so einer radialen Verformung des Schlauchs entgegenwirkt.

Die der vorliegenden Erfindung zugrundeliegenden Aufgaben werden auch gelöst durch ein Verfahren zum Betreiben einer medizinischen Vorrichtung zur lokalen Applikation eines medizinischen Fluids, wobei das medizinische Fluid im Rahmen des Verfahrens nicht an einen menschlichen oder tierischen Körper abgegeben wird, die Vorrichtung aufweisend einen Schlauch mit einer Schlauchwand, wobei die Schlauchwand eine äußere Wandung aus einem ersten Material aufweist, die radial außen liegend angeordnet ist, und die Schlauchwand eine innere Wandung aus einem zweiten Material aufweist, die radial innen liegend angeordnet ist und die eine innere Leitung des Schlauchs begrenzt, wobei der Schlauch mehrere Öffnungen in der Schlauchwand aufweist, wobei die mehreren Öffnungen die innere Leitung des Schlauchs mit der Umgebung des Schlauchs verbinden, das Verfahren aufweisend die folgenden Schritte:
A) Einleiten eines medizinischen Fluids in den Schlauch;
B) Ausüben eines Drucks auf das medizinische Fluid in dem Schlauch;
C) Öffnen der mehreren Öffnungen in der inneren Wandung oder in der äußeren Wandung des Schlauchs durch den auf die mehreren Öffnungen wirkenden Druck des medizinischen Fluids; und
D) Austreiben von medizinischem Fluid durch die geöffneten mehreren Öffnungen.

Die Schritte werden vorzugsweise chronologisch nacheinander durchgeführt.

Nach Schritt D) kann der Druck auf das medizinische Fluid reduziert werden und dadurch die mehreren Öffnungen verschlossen werden.

Es kann vorgesehen sein, dass bei dem Verfahren keine medizinische Behandlung eines menschlichen oder tierischen Körpers erfolgt.

Hiermit wird klargestellt, dass es sich bei dem erfindungsgemäßen Verfahren nicht um ein Verfahren zur Behandlung des menschlichen Körpers handelt.

Des Weiteren kann vorgesehen sein, dass Schritt E) Reduzieren des Drucks auf das medizinische Fluid in dem Schlauch nach Schritt D) und dadurch Schließen der mehreren Öffnungen in der inneren Wandung des Schlauchs oder Verringern des freien Querschnitts der mehreren Öffnungen in der inneren Wandung des Schlauchs.

Hierdurch kann das Verfahren alternierend eingesetzt werden, ohne dass das medizinische Fluid in der inneren Leitung durch rückströmende Fluide kontaminiert wird.

Ferner kann vorgesehen sein, dass vor Schritt A) der Schlauch durch Abschneiden gekürzt wird und ein Verschlusselement in das gerade geschnittene Ende des Schlauchs gesteckt oder geschraubt wird, wobei das Verschlusselement den Schlauch fluiddicht abschließt, bevorzugt fluiddicht und druckdicht verschließt.

Hiermit kann der Schlauch auf einfache Weise auf eine zur Anwendung passende Länge gebracht werden.

Schließlich kann vorgesehen sein, dass das Verfahren mit einer erfindungsgemäßen Vorrichtung durchgeführt wird.

Hierdurch ergeben sich für das Verfahren die zu den jeweiligen Ansprüchen genannten Vorteile.

Der Erfindung liegt die überraschende Erkenntnis zugrunde, dass es mit Hilfe eines Schlauchs mit einer inneren Wandung und einer äußeren Wandung aus unterschiedlichen Materialien gelingt, die in der Schlauchwandung vorhandenen mehreren Öffnungen abhängig vom Druck eines medizinischen Fluids oder abhängig von anderen physikalischen Zustandsgrößen, Wirkungen oder Feldern reversibel zu öffnen und zu schließen, um so temporär ein medizinisches Fluid abzugeben. Durch die Wirkung nur auf die innere Wandung oder nur auf die äußere Wandung des Schlauchs können die mehreren Öffnungen verschlossen oder geöffnet werden. Gleichzeitig werden dabei auf die äußere Wandung keine Kräfte ausgeübt oder eine Verformung der äußeren Form des Schlauchs vermieden. Dadurch bleibt der Schlauch außen formstabil. Eine mechanische Beanspruchung der angrenzenden mit dem Fluid zu behandelnden Oberflächen wird so vermieden. Das hierdurch bereitgestellte Ventil kann so ohne eine Veränderung der äußeren Form des Schlauchs geöffnet und wieder verschlossen werden. Hierdurch kann beispielsweise eine mechanische Reizung eines angrenzenden entzündeten Gewebes verhindert oder zumindest reduziert werden. Insbesondere dann, wenn die mehreren Öffnungen nur dann fluidleitend geöffnet sind, wenn diese durch einen Druck des medizinischen Fluids geöffnet werden, sind die mehreren Öffnungen und damit die Vorrichtung ohne weiteres Einspeisen des medizinischen Fluids geschlossen.

Eine weitere überraschende Erkenntnis ist darin zu sehen, dass der Schlauch problemlos gekürzt werden kann und so dessen Länge an die jeweilige Situation angepasst werden kann. Hierzu muss lediglich das distale Ende des Schlauchs mit einem vorhandenen oder einem neuen Verschlusselement verschlossen werden. Die Vorrichtung ist kostengünstig vollständig oder weitgehend aus Kunststoff herzustellen und kann so als hygienisches Einmalprodukt zur Verfügung gestellt werden. Die mehreren Öffnungen im Schlauch werden dabei so verschlossen, dass im geschlossenen Zustand keine Hinterschneidungen im Zwischenraum zwischen der äußeren Wandung und der inneren Wandung entstehen, in die Gewebe einwachsen könnte und so ein Entfernen der Vorrichtung beziehungsweise des Schlauchs erschweren würde.

Die Vorrichtung besitzt vorzugsweise eine außerhalb des Patienten zu betätigende Ventilfunktion. Die erfindungsgemäße Vorrichtung kann je nach anatomischer Situation des Implantationsortes oder je nach Tiefe des Hohlraums hinsichtlich ihrer Länge durch einfaches mechanisches Kürzen angepasst werden, ohne dass ein Funktionsverlust eintritt.

Der besondere Vorteil der erfindungsgemäßen Vorrichtung besteht darin, dass der medizinische Anwender jedes beliebige medizinische Fluid mit exakt definiertem Volumen applizieren kann. Bei wirkstoffhaltigen Fluiden können ein oder mehrere pharmazeutische Wirkstoffstoffe in dem Fluid in genau vorbestimmten Konzentrationen eingestellt werden. Dadurch ist es möglich, genau definierte Wirkstoffkonzentrationen in unmittelbarer Nähe der Öffnungen der Vorrichtung zu erreichen und damit zu behandeln. Ein weiterer Vorteil der Vorrichtung ist, dass die mehreren Öffnungen im Schlauch nur während der Applikation geöffnet sind und danach verschlossen werden, so dass kein Blut oder Gewebeflüssigkeit und auch kein sich bildendes Bindegewebe in den Zwischenraum zwischen der inneren Wandung und der äußeren Wandung eindringen kann und dort Hinterschneidungen bilden kann, die beim Entfernen der Vorrichtung reißen und dadurch neue Irritationen des gerade behandelten Gewebes verursachen. Zudem werden Verstopfungen der Vorrichtung, insbesondere der Öffnungen im Schlauch, vermieden.

Eine beispielhafte erfindungsgemäße Vorrichtung zur lokalen Applikation von Fluiden mit Ventilfunktion ist zusammengesetzt aus
a) einem plastisch verformbaren Schlauch, wobei der Schlauch in ein distales Teilstück und ein proximales Teilstück unterteilt ist,
b) wobei das distale Teilstück des Schlauchs mindestens zwei Öffnungen in der Mantelfläche besitzt, welche den Innenraum des Schlauchs mit der Umgebung verbinden, wobei die Summe der Querschnittsflächen der Öffnungen gleich oder kleiner dem Innenquerschnitt des Schlauchs sind,
c) das proximale Teilstück des Schlauchs keine Öffnungen in der Mantelfläche enthält, die den Innenraum des Schlauchs mit der Umgebung verbinden,
d) einem manuell einzusetzenden Verschlusselement, welches ein distales Ende des distalen Teilstücks des Schlauchs flüssigkeits- und gasdicht verschließt, und
e) einem distalen Schlauchende des proximalen Teilstücks des Schlauchs, das mit einem diskontinuierlich oder kontinuierlich Wirkstofflösung fördernden Wirkstoffreservoir flüssigkeitsdicht verbindbar oder verbunden ist.

Das Verschlusselement ist beispielsweise zusammengesetzt aus einem ersten rotationssymmetrischer Körper mit Außengewinde, wobei das Außengewinde einen größeren Außendurchmesser besitzt als der Innendurchmesser des Schlauchs, und aus einem zweiten rotationssymmetrischen Körper mit einem Außendurchmesser kleiner oder gleich dem Außendurchmesser des äußeren Schlauchs, wobei die axiale Erstreckung des zweiten rotationssymmetrischen Körpers mindestens 5 mm ist, und wobei der erste rotationssymmetrische Körper axial mit dem zweiten rotationssymmetrischen Körper verbunden ist.

In einer weiteren alternativen Ausgestaltungsform ist ein beispielhaftes Verschlusselement zusammengesetzt aus einem ersten rotationssymmetrischer Körper mit am Umfang umlaufenden Stegen, wobei die Stege einen größeren Außendurchmesser besitzen als der Innendurchmesser des inneren Schlauchs, und aus einem zweiten rotationssymmetrischen Körper mit einem Außendurchmesser kleiner oder gleich dem Außendurchmesser des äußeren Schlauchs, wobei die axiale Erstreckung des zweiten rotationssymmetrischen Körpers mindestens 5 mm ist, und wobei der erste rotationssymmetrische Körper axial mit dem zweiten rotationssymmetrischen Körper verbunden ist.

Im Folgenden werden weitere Ausführungsbeispiele der Erfindung anhand von zwölf schematisch dargestellten Figuren erläutert, ohne jedoch dabei die Erfindung zu beschränken.

Dabei zeigt:
Figur 1: eine schematische perspektivische Ansicht einer beispielhaften ersten erfindungsgemäßen Vorrichtung zur lokalen Applikation eines Fluids;
Figur 2: eine schematische Seitenansicht auf ein distales Ende der Vorrichtung nach Figur 1;
Figur 3: eine schematische Seitenansicht auf eine proximal angeordnete Fördereinrichtung enthaltend einen Behälter der Vorrichtung nach den Figuren 1 und 2;
Figur 4: eine schematische perspektivische Teilschnittansicht der Fördereinrichtung der Vorrichtung nach den Figuren 1 bis 3;
Figur 5: eine schematische Teilschnittaufsicht der Fördereinrichtung nach Figur 4 im gespannten Zustand;
Figur 6: eine schematische Teilschnittaufsicht der Fördereinrichtung nach Figur 4 im entspannten Zustand;
Figur 7: mehrere schematische perspektivische Ansichten von distalen Schlauchteilstücken zweier erfindungsgemäßer Vorrichtungen im geöffneten und verschlossenen Zustand;
Figur 8: mehrere schematische perspektivische Ansichten der distalen Schlauchteilstücke der beiden Vorrichtungen nach Figur 7 im geöffneten und verschlossenen Zustand;
Figur 9: vier schematische Querschnittansichten der distalen Schlauchteilstücke der beiden Vorrichtungen nach den Figuren 7 und 8 mit geöffneten Öffnungen;
Figur 10: vier schematische Querschnittansichten der distalen Schlauchteilstücke der beiden Vorrichtungen nach den Figuren 7 bis 9 mit geschlossenen Öffnungen;
Figur 11: zwei schematische perspektivische Ansichten einer beispielhaften weiteren erfindungsgemäßen Vorrichtung zur lokalen Applikation eines Fluids; und
Figur 12: vier schematische Detailansichten eines Anschlagelements des Ausführungsbeispiels nach Figur 11.

In den Figuren und in der folgenden Beschreibung zu den anhand der Figuren erläuterten Ausführungsbeispiele der vorliegenden Erfindung werden teilweise für unterschiedliche Ausführungsbeispiele die gleichen Bezugszeichen bei gleichen oder gleichartigen Teilen verwendet, um die Vergleichbarkeit der Ausführungsbeispiele und die Lesbarkeit zu vereinfachen.

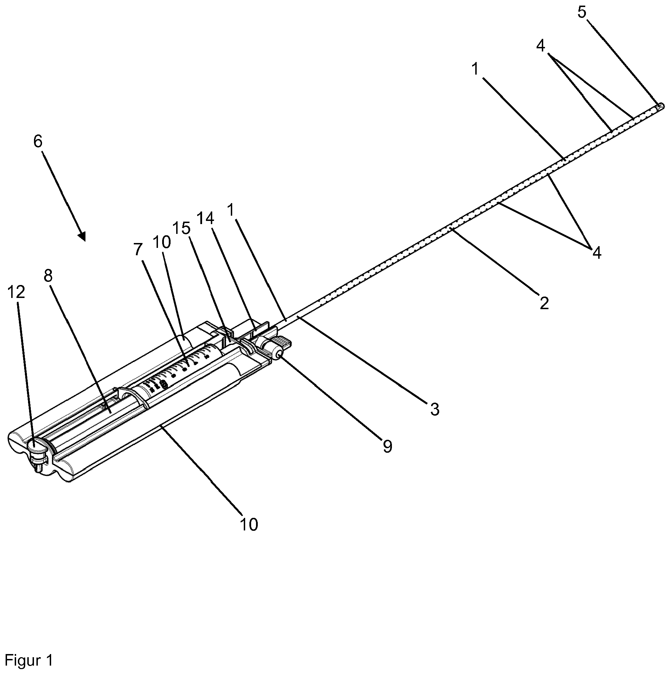

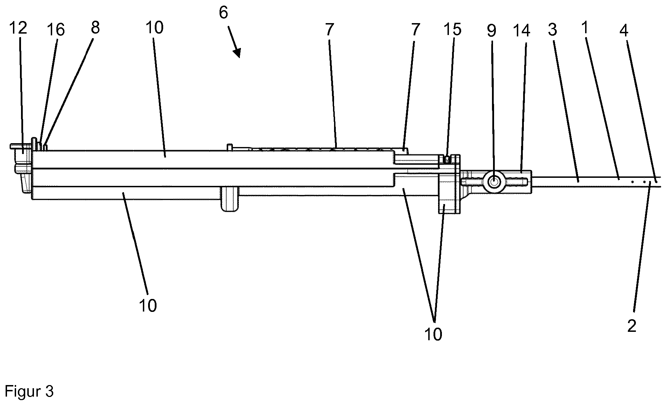

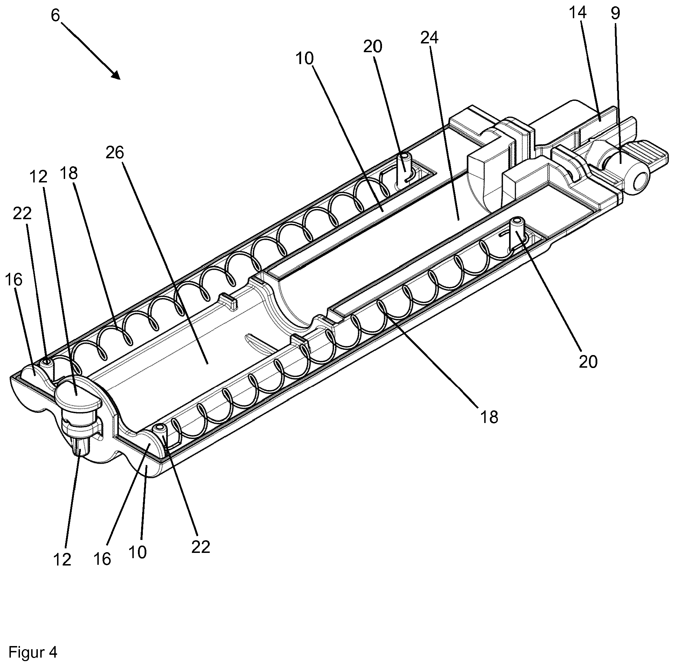

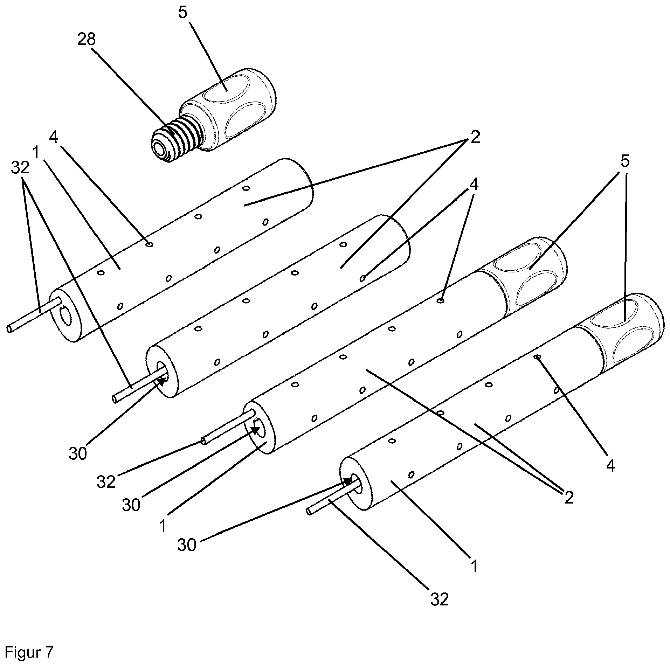

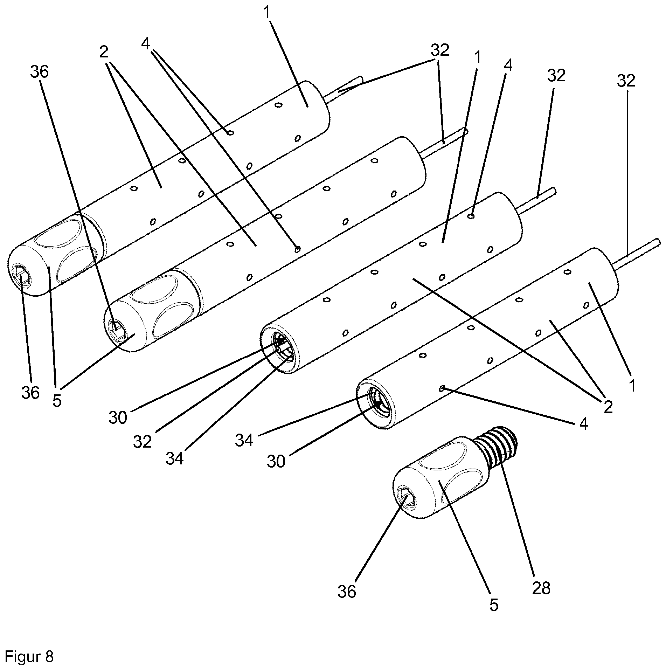

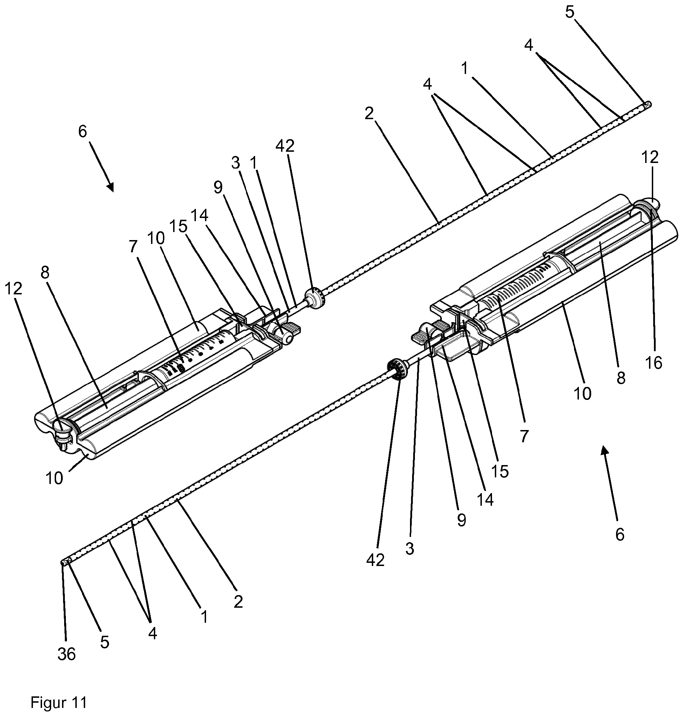

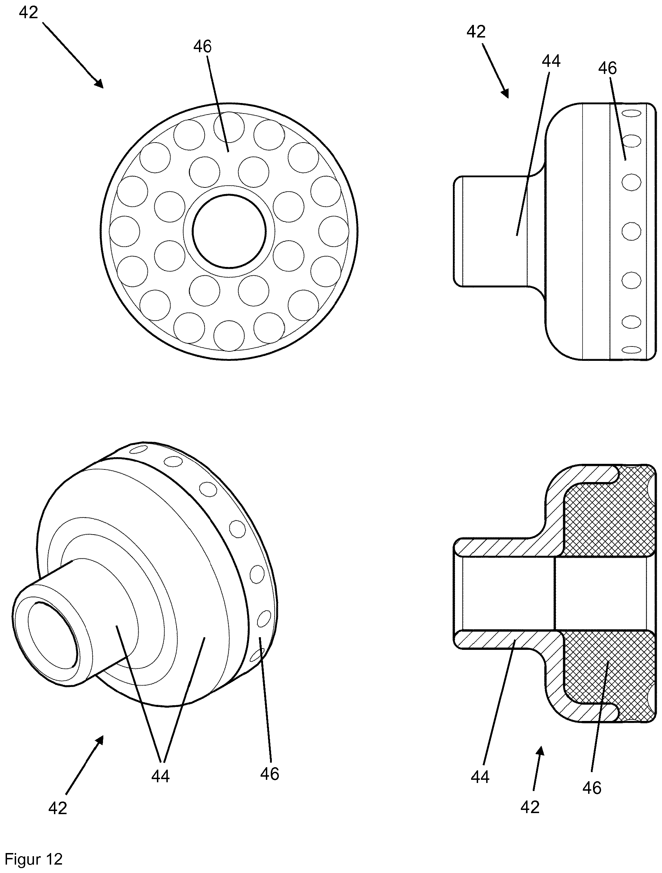

Die Figuren 1 bis 6 zeigen eine erste beispielhafte erfindungsgemäße Vorrichtung und Teile davon in unterschiedlichen Darstellungen. Die Figuren 7 bis 10 zeigen offene und geschlossene distale Schlauchendstücke als Teile von zwei unterschiedlichen erfindungsgemäßen Ausführungsbeispielen. In den Figuren 11 und 12 ist ein weiteres erfindungsgemäßes Ausführungsbeispiel der vorliegenden Erfindung gezeigt.

Die erste beispielhafte erfindungsgemäße Vorrichtung, die in den Figuren 1 bis 6 gezeigt ist, hat an der vorderen distalen Seite (in Figur 1 rechts oben) einen Schlauch 1 mit einem distalen Teilstück 2 und einem proximalen Teilstück 3. In dem distalen Teilstück 2 des Schlauchs 1 können mehrere durch die Schlauchwandung gehende Öffnungen 4 angeordnet sein, die bis in eine innere Leitung 30 (in den Figuren 1 bis 6 nicht zu sehen, aber in den Figuren 7 bis 10) des Schlauchs 1 reichen. Der Schlauch 1 kann plastisch verformbar sein, so dass die Form des Schlauchs 1 an die Form eines zu spülenden Hohlraums angepasst werden kann. Das proximale Teilstück 3 des Schlauchs 1 weist keine Öffnungen in der Schlauchwand auf. Damit wird sichergestellt, dass ein medizinisches Fluid (nicht gezeigt), das mit einer solchen Vorrichtung appliziert wird, nur innerhalb des zu spülenden Hohlraums austritt.

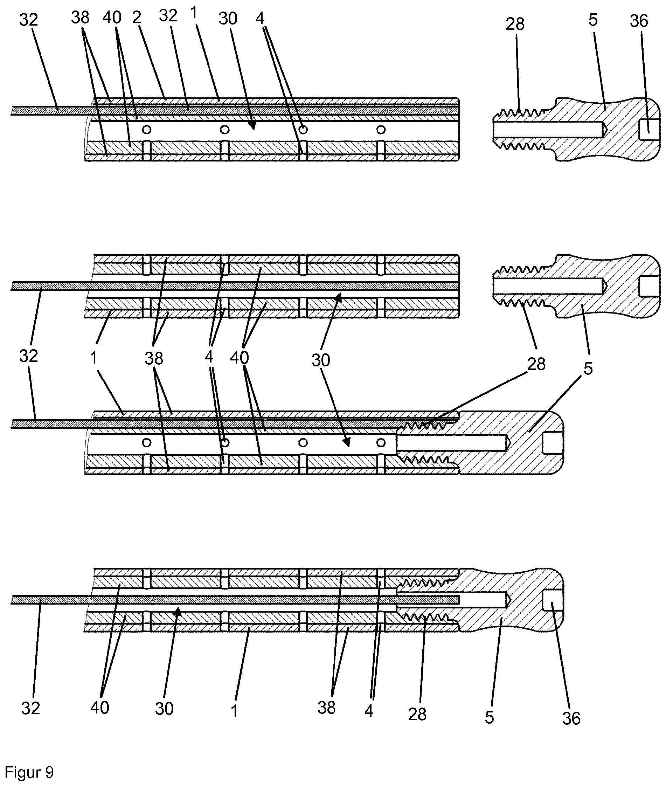

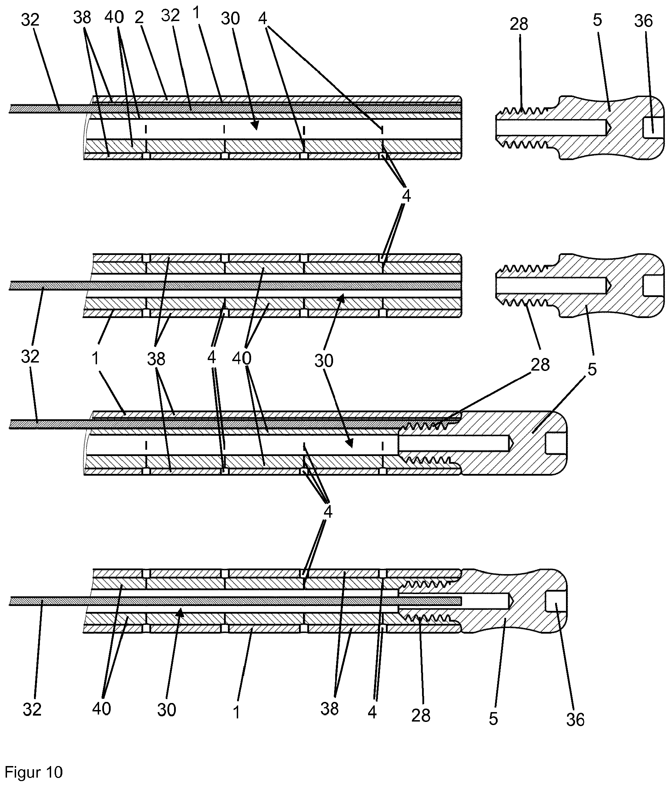

Das distale Teilstück 2 des Schlauchs 1 kann an einem distalen Ende mit einem Verschlusselement 5 fluiddicht und druckdicht verschlossen sein. Der Schlauch 1 weist eine innere Wandung 40 und eine äußere Wandung 38 auf (in den Figuren 1 bis 6 nicht zu sehen, aber analog den Figuren 9 und 10 aufgebaut), wobei die äußere Wandung 38 die innere Wandung 40 umschließt, bevorzugt koaxial umschließt. Die Öffnungen 4 reichen durch die äußere Wandung 38 und die innere Wandung 40. Im Inneren des Schlauchs 1 ist die innere Leitung 30 vorzugsweise durch die innere Wandung 40 begrenzt. Die Öffnungen 4 können die innere Leitung 30 mit der Umgebung des Schlauchs 1 flüssigkeitsdurchlässig verbinden. Die innere Wandung 40 kann aus einem elastisch verformbaren Material wie einem gummielastischen Polymer, insbesondere Polyurethan, bestehen. Die äußere Wandung 38 kann aus einem nicht gummielastischen thermoplastischen Polymer, insbesondere aus Polyamid, bestehen. Dadurch ist die innere Wandung 40 elastisch verformbar, während die äußere Wandung 38 gegenüber einer radialen Ausdehnung des Schlauchs 1 weitgehend formstabil ist. Das Material für die äußere Wandung 38 kann so gewählt werden, dass eine Verformung der Längsachse des Schlauchs 1 möglich ist, während eine radiale Ausdehnung des Schlauchs 1 aufgrund eines inneren Drucks in der inneren Leitung 30 nicht möglich ist oder nur maximal eine fünfprozentige relative axiale Dehnung möglich ist.

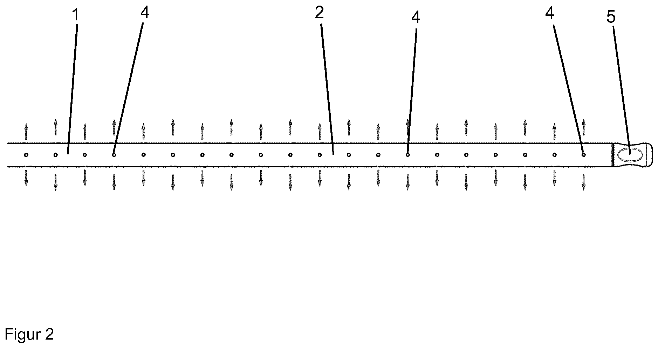

Die Öffnungen 4 können durch die innere Wandung 40 des Schlauchs 1 gestochen sein, so dass das Material der inneren Wandung 40 nicht ausgestanzt wird. Ähnlich wie bei gummielastischen Membranen für Behälter zum Aufziehen von Spritzen können sich dadurch die Öffnungen 4 in der inneren Wandung 40 verschließen (siehe Figur 10). Wenn in der inneren Leitung 30 ein Druck auf ein Fluid darin ausgeübt wird, öffnet dieser Druck die Öffnungen 4 in den inneren Wandungen 40 des Schlauchs 1 (siehe Figur 9) und das Fluid kann aus den Öffnungen 4 austreten, so wie das in Figur 2 durch die Pfeile angedeutet ist.

Die Öffnungen 4 können radial verteilt und entlang der gesamten Länge des distalen Teilstücks 2 verteilt sein. Die in dem Ausführungsbeispiel angedeuteten Öffnungen 4 in vier axialen Richtungen sind dabei nur beispielhaft zu verstehen. Der Schlauch 1 kann auch an die Größe des zu spülenden Hohlraums angepasst werden, indem er am distalen Teilstück 2 durch abschneiden gekürzt wird. Mit dem Verschlusselement 5 kann das neue geschnittene distale Schlauchende abgedichtet werden. Hierzu kann das Verschlusselement 5 in die innere Leitung 30 eingesteckt oder eingeschraubt werden.

An der proximalen Seite der Vorrichtung kann eine Fördereinrichtung 6 angeordnet sein. In die Fördereinrichtung 6 kann ein Behälter 7 in Form einer Spritze mit einem Kolben 8 zum Auspressen des Inhalts der Spritze eingesetzt werden beziehungsweise eingesetzt sein. Der Kolben 8 kann in axialer Richtung in der Spritze verschiebbar angeordnet sein und fluiddicht gegen die Innenwand des Behälters 7 abgedichtet sein. Die Fördereinrichtung 6 kann ein Gehäuse 10 aus einem Plastik aufweisen, das das Innere der Fördereinrichtung 6 vollständig oder teilweise nach außen verschließen kann. Ein Sicherungsbolzen 12 kann in eine Öffnung am proximalen Ende des Gehäuses 10 eingesteckt sein.

Auf der distalen Seite der Fördereinrichtung 6 kann eine Halterung 14 zur Befestigung des proximalen Endes des Schlauchs 1 angeordnet sein. Hierzu kann an dem Schlauch 1 eine Halterungsscheibe 15 befestigt sein, die in die Halterung 14 eingreifen kann.

In der Fördereinrichtung 6 kann eine Förderplatte 16 zum Einpressen des Kolbens 8 in den Behälter 7 angeordnet sein. Mit dem Sicherungsbolzen 12 kann die Förderplatte 16 gegen das Gehäuse 10 arretiert werden. Hierzu kann eine Öse an der proximalen Seite der Förderplatte 16 aus dem Gehäuse 10 der Fördereinrichtung 6 ragen und durch Einstecken des Sicherungsbolzens 12 die Förderplatte 16 gegen das Gehäuse 10 arretiert werden. Die Förderplatte 16 kann von zwei gespannten Federn 18 angetrieben werden. Die beiden Federn 18 stellen ein Energiespeicherelement dar, in denen zumindest die Energie gespeichert ist, die zum Auspressen eines medizinischen Fluids aus dem Behälter 7 und durch den Schlauch 1 und durch die Öffnungen 4 des Schlauchs 1 notwendig ist.

Die Federn 18 können an ihren distalen Enden mit Stiften 20 am Gehäuse 10 befestigt sein. An ihren proximalen Enden können die Federn 18 mit Stiften 22 an der Förderplatte 16 befestigt sein. Die Federn 18 können so zwischen den Stiften 20 und den Stiften 22 gespannt sein.

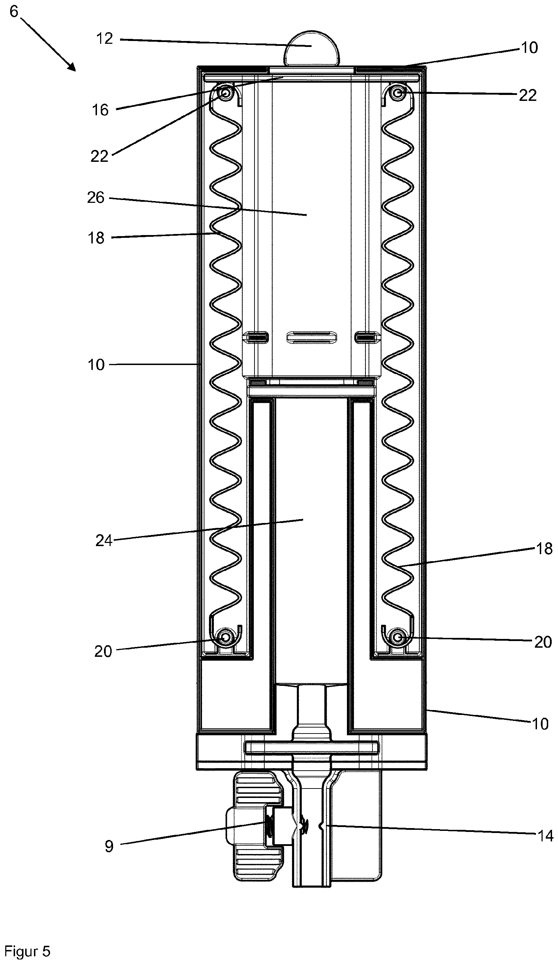

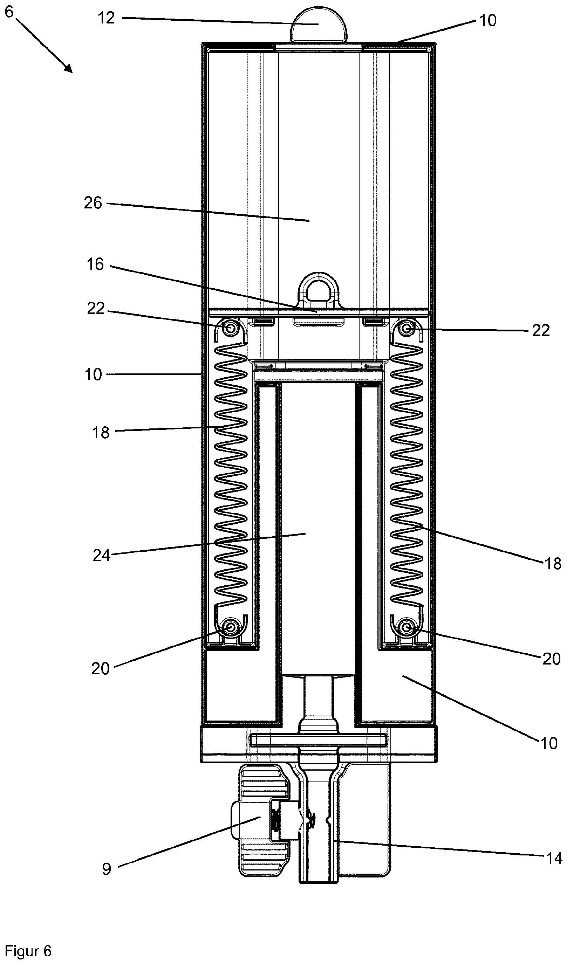

Im Inneren des Gehäuses 10 kann eine Aufnahme 24 für den Behälter 7 und ein Hubraum 26 für den Kolben 8 ausgeformt sein. Der Behälter 7 kann durch die Form der Aufnahme 24 in der Fördereinrichtung 6 fixiert werden. Die Förderplatte 16 kann auf diese Weise von den Federn 18 vom proximalen Ende bis zum distalen Ende des Hubraums 26 gezogen werden (siehe Figuren 5 und 6). Der Kolben 8 kann in der Fördereinrichtung 6 mit der Förderplatte 16 angetrieben durch die Federn 18 in den Behälter 7 gepresst werden, wenn der Sicherungsbolzen 12 entfernt wurde und das Ventilelement 9 geöffnet ist. Dadurch kann ein in dem Behälter 7 enthaltenes medizinisches Fluid aus dem Behälter 7 und durch den Schlauch 1 und die Öffnungen 4 des Schlauchs 1 ausgepresst werden. Mit dem auf das medizinische Fluid wirkenden Druck können dabei die Öffnungen 4 in der inneren Wandung des Schlauchs 1 geöffnet werden.

Die in den Figuren 7 bis 10 gezeigten Schläuche 1 können ohne weiteres in der nach den Figuren 1 bis 6 gezeigten Vorrichtung angewendet werden und so die Figuren 7 bis 10 als Detailzeichnungen des ersten Ausführungsbeispiels verstanden werden. Die beiden Varianten der Schläuche 1 nach den Figuren 7 bis 10 unterscheiden sich durch die Anordnung eines Metalldrahts 32, der entweder in einer inneren Leitung 30 des Schlauchs 1 angeordnet sein kann oder in der Schlauchwand. Der Schlauch 1 kann in beiden Fällen an seinem distalen Ende mit einem Verschlusselement 5 verschließbar sein. Das Verschlusselement 5 weist einen vorstehenden zylindrischen Fortsatz mit einem Außengewinde 28 auf. Mit dem Außengewinde 28 kann das Verschlusselement 5 in die offene innere Leitung 30 des Schlauchs 1 eingeschraubt werden. Das Verschlusselement 5 besteht vorzugsweise aus Metall. Das Außengewinde 28 kann sich ein passendes Innengewinde 34 in die Innenwand des Schlauchs 1 schneiden. Dadurch kann der Schlauch 1 an seinem distalen Ende flüssigkeitsdicht und druckdicht verschlossen werden. In dem distalen Kopf des Verschlusselements 5 kann ein Innensechskant 36 angeordnet sein oder es kann ein anderer Schraubkopf vorgesehen sein, um das Verschlusselement 5 bequemer in den Schlauch 1 einschrauben zu können.

Analog dem ersten Ausführungsbeispiel nach den Figuren 1 bis 6 weist der Schlauch 1 eine äußere Wandung 38 und eine innere Wandung 40 auf (siehe Figuren 9 und 10). Die äußere Wandung 38 umschließt die innere Wandung 40 vorzugsweise vollständig. Die Materialien zur Ausbildung der äußeren Wandung 38 und der inneren Wandung 40 können dabei analog dem ersten Ausführungsbeispiel so gewählt werden, dass die äußere Wandung 38 formstabil ist und die innere Wandung 40 gummielastisch ist. Mehrere Öffnungen 4 reichen durch die äußere Wandung 38 und die innere Wandung 40 bis zur inneren Leitung 30 des Schlauchs 1.

Wenn kein Druck eines medizinischen Fluids in der inneren Leitung 30 des Schlauchs 1 wirkt, kann das Material der inneren Wandung 40 entspannen. Die Öffnungen 4 werden dadurch in der inneren Wandung 40 fluiddicht verschlossen (siehe Figur 10). Die Öffnungen 4 sind in Figur 10 in der inneren Wandung 40 als Schlitze zu erkennen. Durch einen Druck auf das medizinische Fluid können die Öffnungen 4 in der inneren Wandung 40 geöffnet werden. Dabei wird der Schlauch 1 nicht radial ausgedehnt, da die äußere Wandung 38 die Kräfte aufnehmen kann. Das medizinische Fluid kann durch die geöffneten Öffnungen 4 austreten.

Mit Hilfe des Metalldrahts 32 kann der Schlauch 1 bei beiden Ausführungen plastisch verformt werden und hält seine Form.

Die Figuren 11 und 12 zeigen eine weitere Ausführungsform der vorliegenden Erfindung. Diese Ausführungsform entspricht den Ausführungen nach den Figuren 1 bis 10, außer das zusätzlich ein Versiegelungselement 42 auf den Schlauch 1 gesteckt ist. Die Bezugszeichen und der Aufbau der Ausführungsform nach den Figuren 11 und 12 entsprechen also denen der vorhergehenden Ausführungen. Der innere Aufbau des Schlauchs 1 mit einer inneren Wandung 40 und einer die innere Wandung 40 koaxial umgebenden äußeren Wandung 38, wobei eine Wandung durch den Druck eines medizinischen Fluids elastisch verformbar ist, während die andere Wandung radiale Formstabilität bewirkt, ist auch bei dieser Ausführungsform realisiert.

Bevorzugt kann das Versiegelungselement 42 auf dem Schlauch 1 axial (bezogen auf die zylindrische Schlauchachse) verschiebbar sein. Gemäß einer bevorzugten Ausführung ist das Versiegelungselement 42 am distalen Ende des proximalen Teilstücks 3 des Schlauchs 1 angeordnet. Dann überdeckt das Versiegelungselement 42 keine der Öffnungen 4 und es ist für den Anwender erkennbar, wo das proximale Teilstück 3 endet beziehungsweise wo das angrenzende distale Teilstück 2 des Schlauchs 1 beginnt.

Das Versiegelungselement 42 kann hülsenförmig geformt sein und eine äußere Hülsenform 44 und eine distale Schwammhülse 46 aufweisen. Die Schwammhülse 46 kann mit einer antibiotischen und/oder desinfizierenden Lösung getränkt sein. Das Versiegelungselement 42 kann auf eine Eintrittsöffnung eines Körpers geschoben werden und dadurch einen Keimeintritt in die Eintrittsöffnung verhindern.

Das Verschlusselement 5 kann bei allen Ausführungen nach Kürzen des Schlauchs 1 vom medizinischen Anwender einfach in das distale (erste) Schlauchende des Schlauchs 1 eingeschraubt oder eingesteckt werden. Dadurch wird die innere Leitung 30 des Schlauchs 1 verschlossen.

Als das zu applizierende medizinische Fluid kann je nach Anwendung beispielsweise eine desinfizierende Flüssigkeit oder eine wässrige Lösung umfassend wenigstens ein Antibiotikum und/oder wenigstens ein Antimykotikum verwendet werden. Ferner kann das medizinische Fluid auch wenigstens ein Zytostatikum und/oder wenigstens ein Chemotherapeutikum enthalten.

Für eine medizinische Anwendung der erfindungsgemäßen Vorrichtungen können der Schlauch 1 und vorzugsweise auch die Verschlusselemente 5 aus biokompatiblen Materialien aufgebaut werden, in denen Röntgenopaker enthalten sind, so dass die Lage des Schlauchs 1 und gegebenenfalls des Verschlusselements 5 mit bildgebenden Röntgenverfahren bestimmbar ist.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Schlauch
- 2: Distales Teilstück
- 3: Proximales Teilstück
- 4: Öffnung
- 5: Verschlusselement
- 6: Fördereinrichtung
- 7: Behälter
- 8: Kolben
- 9: Ventilelement
- 10: Gehäuse
- 12: Verschlussbolzen
- 14: Halterung
- 15: Halterungsscheibe
- 16: Förderplatte
- 18: Feder
- 20: Stift
- 22: Stift
- 24: Aufnahme für Behälter
- 26: Hubraum für Kolben
- 28: Außengewinde
- 30: Innere Leitung des Schlauchs
- 32: Metalldraht
- 34: Innengewinde
- 36: Innensechskant
- 38: Äußere Wandung
- 40: Innere Wandung
- 42: Versiegelungselement
- 44: Hülsenform
- 46: Schwammhülse

## Patentansprüche

1. Vorrichtung zur lokalen Applikation eines medizinischen Fluids aufweisend einen Schlauch (1), der flexibel verformbar ist und der eine Schlauchwand aufweist,
wobei die Schlauchwand eine äußere Wandung (38) aus einem ersten Material aufweist, die radial außen liegend angeordnet ist, und die Schlauchwand eine innere Wandung (40) aus einem zweiten Material aufweist, die radial innen liegend angeordnet ist und die eine innere Leitung (30) des Schlauchs (1) begrenzt,
wobei der Schlauch (1) in einem distalen Teilstück (2) des Schlauchs (1) mehrere Öffnungen (4) in der Schlauchwand aufweist, wobei die mehreren Öffnungen (4) die innere Leitung (30) des Schlauchs (1) mit der Umgebung des Schlauchs (1) verbinden, wobei das distale Teilstück (2) des Schlauchs (1) von einem distalen Ende des Schlauchs (1) begrenzt ist, wobei
die mehreren Öffnungen (4) in der inneren Wandung (40) abhängig von einer auf das zweite Material wirkenden physikalischen Größe, insbesondere abhängig von einem von dem medizinischen Fluid auf das zweite Material wirkenden Druck, reversibel fluiddicht verschließbar sind oder
die mehreren Öffnungen (4) in der äußeren Wandung (38) abhängig von einer auf das erste Material wirkenden physikalischen Größe, insbesondere abhängig von einem von dem medizinischen Fluid auf das erste Material wirkenden Druck, reversibel fluiddicht verschließbar sind,
wobei der Schlauch (1) in einem proximalen Teilstück (3) des Schlauchs (1) keine Öffnungen (4) in der Schlauchwand aufweist, die die innere Leitung (30) des Schlauchs (1) mit der Umgebung des Schlauchs (1) flüssigkeitsdurchlässig verbinden,
die Vorrichtung ferner aufweisend ein Verschlusselement (5), mit dem der Schlauch (1) an dem distalen Ende des Schlauchs (1) flüssigkeitsdicht verschlossen ist oder verschließbar ist, wobei das Verschlusselement (5) manuell in das distale Ende des Schlauchs (1) einsetzbar ist,
wobei ein proximales Ende des Schlauchs (1) derart mit einem Behälter (7) für das medizinische Fluid flüssigkeitsdurchlässig verbunden ist oder verbindbar ist, dass das medizinische Fluid aus dem Behälter (7) durch das proximale Ende des Schlauchs (1) in die innere Leitung (30) des Schlauchs (1) drückbar ist und durch die mehreren Öffnungen (4) an die Umgebung des Schlauchs (1) herausdrückbar ist.

2. Vorrichtung nach Anspruch 1, **dadurch gekennzeichnet, dass**
die innere Leitung (30) des Schlauchs (1) an einer proximalen Öffnung im proximalen Ende des Schlauchs (1) beginnt und an einer distalen Öffnung im distalen Ende des Schlauchs (1) endet, wobei die distale Öffnung des Schlauchs (1) durch das Verschlusselement (5) verschlossen ist oder verschließbar ist, und/oder
die Vorrichtung den Behälter (7) für das medizinische Fluid aufweist, wobei bevorzugt der Behälter (7) einen Hohlzylinder mit einem axial im Hohlzylinder verschiebbaren Kolben (8) aufweist, der ein erstes Ende des Hohlzylinders verschließt, wobei der Hohlzylinder an einem dem ersten Ende gegenüberliegenden Ende eine Austragsöffnung aufweist, die mit dem proximalen Ende des Schlauchs (1) verbunden oder verbindbar ist, bevorzugt über ein manuell bedienbares Ventilelement (9) zur Regulierung der Strömungsgeschwindigkeit des medizinischen Fluids mit dem proximalen Ende des Schlauchs (1) verbunden oder verbindbar ist, wobei vorzugsweise
in dem Behälter (7) ein medizinisches Fluid, insbesondere ein pharmazeutisches Fluid, enthalten ist.

3. Vorrichtung nach einem der vorangehenden Ansprüche, **dadurch gekennzeichnet, dass**
die Vorrichtung eine Fördereinrichtung (6) aufweist, mit der das medizinische Fluid aus dem verbundenen oder verbindbaren Behälter (7) in den Schlauch (1), durch die innere Leitung (30) des Schlauchs (1) und durch die mehreren Öffnungen (4) in die Umgebung des Schlauchs (1) zu drücken ist, wobei vorzugsweise
die Fördereinrichtung (6) ein Energiespeicherelement, insbesondere zumindest eine gespannte Feder (18), aufweist, wobei die Fördereinrichtung (6) mit Energie aus dem Energiespeicherelement antreibbar ist, wobei besonders bevorzugt mit dem Energiespeicherelement ein Kolben (8) in einem Hohlzylinder als der Behälter (7) in Richtung einer gegenüberliegenden Austragsöffnung anzutreiben ist.

4. Vorrichtung nach einem der vorangehenden Ansprüche, **dadurch gekennzeichnet, dass**
die äußere Wandung (38) und die innere Wandung (40) miteinander fest verbunden sind, bevorzugt vollflächig miteinander verbunden sind, und
die Summe der freien Querschnittsflächen aller der mehreren Öffnungen (4) maximal so groß ist wie der freie Querschnitt der inneren Leitung (30).

5. Vorrichtung nach einem der vorangehenden Ansprüche, **dadurch gekennzeichnet, dass**
die mehreren Öffnungen (4) in der äußeren Wandung (38) der Schlauchwand unabhängig vom Druck des medizinischen Fluids offen sind, während die mehreren Öffnungen (4) in der inneren Wandung (40) der Schlauchwand ohne Druckbeaufschlagung durch das medizinische Fluid verschlossen sind und durch einen Druck auf das medizinische Fluid flüssigkeitsdurchlässig zu öffnen sind und/oder
die äußere Wandung (38) der Schlauchwand einen über die innere Wandung (40) der Schlauchwand vermittelten Druck des medizinischen Fluids in der inneren Leitung (30) aufnimmt, ohne sich dabei radial um mehr als 5% auszudehnen, bevorzugt ohne sich dabei radial um mehr als 1% auszudehnen, oder
sich der Schlauch (1) bei einem Innendruck von 500 kPa gegenüber Normaldruck sich radial um maximal 5 Prozent ausdehnt, bevorzugt um maximal 1 Prozent ausdehnt.

6. Vorrichtung nach einem der vorangehenden Ansprüche, **dadurch gekennzeichnet, dass**
das Verschlusselement (5) einen konischen oder zylindrischen Vorsprung aufweist, der in den Schlauch (1) gesteckt oder geschraubt ist, so dass der Schlauch (1) im Bereich des distalen Endes des äußeren Schlauchs (1) durch den Vorsprung derart gespannt ist, dass der Schlauch (1) am distalen Ende des Schlauchs (1) fluiddicht verschlossen ist, wobei vorzugsweise der konische oder zylindrische Vorsprung Stege an der Außenseite des Vorsprungs aufweist oder der konische oder zylindrische Vorsprung ein Außengewinde (28) aufweist, wobei das Außengewinde (28) oder der konische oder zylindrische Vorsprung einen größeren Außendurchmesser aufweist als der Innendurchmesser des Schlauchs (1).

7. Vorrichtung nach einem der vorangehenden Ansprüche, **dadurch gekennzeichnet, dass**
die mehreren Öffnungen (4) im distalen Teilstück (2) des Schlauchs (1) in der äußeren Wandung (38) einen Durchmesser von maximal 500 µm, bevorzugt von maximal 250 µm und besonders bevorzugt von maximal 100 µm haben, und/oder der Schlauch (1) aus einem koaxialen Koextrudat gebildet ist, wobei die innere Wandung (40) aus einem gummielastischen Polymer, insbesondere Polyurethan oder einem schwach vernetzten Polymer, besteht und die äußere Wandung (38) aus einem nicht gummielastischen thermoplastischen Polymer oder aus einem stark vernetzten Polymer besteht, insbesondere aus Polyamid besteht.

8. Vorrichtung nach einem der vorangehenden Ansprüche, **dadurch gekennzeichnet, dass**
zumindest am distalen Ende des Schlauchs (1) und/oder im Verschlusselement (5) ein röntgenopakes Material im Schlauch (1) enthalten ist, bevorzugt im distalen Teilstück (2) des Schlauchs (1) und/oder im Verschlusselement (5) ein röntgenopakes Material enthalten ist, besonders bevorzugt auf der gesamten Länge des distalen Teilstücks (2) des Schlauchs (1) und im Verschlusselement (5) enthalten ist oder auf der gesamten Länge des Schlauchs (1) und im Verschlusselement (5) enthalten ist, und/oder zumindest ein Metalldraht (32), zumindest eine Metallspirale und/oder zumindest ein Metallnetz in der inneren Leitung (30) des Schlauchs (1) und/oder in der Schlauchwand des Schlauchs (1) angeordnet ist oder sind, wobei der zumindest eine Metalldraht (32), die zumindest eine Metallspirale und/oder das zumindest ein Metallnetz bevorzugt entlang der gesamten Länge des Schlauchs (1) angeordnet sind.

9. Vorrichtung nach einem der vorangehenden Ansprüche, **dadurch gekennzeichnet, dass**
das Verschlusselement (5) die folgenden Merkmale aufweist:
einen rotationssymmetrischen ersten Körper mit Außengewinde (28) oder mit am Umfang umlaufenden Stegen, wobei das Außengewinde (28) oder die Stege einen größeren Außendurchmesser besitzt als der Innendurchmesser des Schlauchs (1),
einen rotationssymmetrischen zweiten Körper mit einem Außendurchmesser kleiner oder gleich dem Außendurchmesser des Schlauchs (1), wobei die axiale Erstreckung des zweiten Körpers mindestens 5 mm ist,
wobei der rotationssymmetrische erste Körper axial mit dem rotationssymmetrischen zweiten Körper verbunden ist, und/oder
das Verschlusselement (5) in das distale Ende des Schlauchs (1) eingeschraubt oder eingepresst ist und den freien Leitungsquerschnitt der inneren Leitung (30) des Schlauchs (1) am distalen Ende vollständig flüssigkeitsdicht und druckdicht verschließt.

10. Vorrichtung nach einem der vorangehenden Ansprüche, **dadurch gekennzeichnet, dass**
der Schlauch (1) plastisch verformbar ist und einen Außendurchmesser kleiner oder gleich 7 mm aufweist, bevorzugt einen Außendurchmesser zwischen 2 mm und 4 mm, und
das erste Material eine größere Shore A-Härte hat als das zweite Material, wobei bevorzugt das erste Material eine Shore A-Härte von mehr als 60 und das zweite Material eine Shore A-Härte von weniger als 60 hat, und/oder die mehreren Öffnungen (4) in der inneren Wandung (40) bei Druckbeaufschlagung mit einem hydrostatischen Druck von 500 kPa durch das medizinische Fluid einen um den Faktor zwei oder mehr größeren freien Querschnitt aufweisen als ohne Druckbeaufschlagung, wobei vorzugsweise
die mehreren Öffnungen (4) in der inneren Wandung (40) oder der äußeren Wandung (38) schlitzförmig geformt sind.

11. Vorrichtung nach einem der vorangehenden Ansprüche, **dadurch gekennzeichnet, dass**
die mehreren Öffnungen (4) durch eine elastische Deformation des zweiten Materials reversibel zu öffnen sind, während die mehreren Öffnungen (4) im ersten Material offen bleiben, wobei vorzugsweise das erste Material derart formfest ist, dass die äußere Wandung (38) zumindest einen Teil der durch die elastische Verformung des zweiten Materials verursachten Kräfte aufnimmt und so einer radialen Verformung des Schlauchs (1) entgegenwirkt.

12. Verfahren zum Betreiben einer medizinischen Vorrichtung zur lokalen Applikation eines medizinischen Fluids, wobei das medizinische Fluid im Rahmen des Verfahrens nicht an einen menschlichen oder tierischen Körper abgegeben wird, die Vorrichtung aufweisend einen Schlauch (1) mit einer Schlauchwand, wobei die Schlauchwand eine äußere Wandung (38) aus einem ersten Material aufweist, die radial außen liegend angeordnet ist, und die Schlauchwand eine innere Wandung (40) aus einem zweiten Material aufweist, die radial innen liegend angeordnet ist und die eine innere Leitung (30) des Schlauchs (1) begrenzt, wobei der Schlauch (1) mehrere Öffnungen (4) in der Schlauchwand aufweist, wobei die mehreren Öffnungen (4) die innere Leitung (30) des Schlauchs (1) mit der Umgebung des Schlauchs (1) verbinden, das Verfahren aufweisend die folgenden Schritte:
A) Einleiten eines medizinischen Fluids in den Schlauch (1);
B) Ausüben eines Drucks auf das medizinische Fluid in dem Schlauch (1);
C) Öffnen der mehreren Öffnungen (4) in der inneren Wandung (40) oder in der äußeren Wandung (38) des Schlauchs (1) durch den auf die mehreren Öffnungen (4) wirkenden Druck des medizinischen Fluids; und
D) Austreiben von medizinischem Fluid durch die geöffneten mehreren Öffnungen (4).

13. Verfahren nach Anspruch 12, **gekennzeichnet durch**
Schritt E) Reduzieren des Drucks auf das medizinische Fluid in dem Schlauch (1) nach Schritt D) und dadurch Schließen der mehreren Öffnungen (4) in der inneren Wandung (40) des Schlauchs (1) oder Verringern des freien Querschnitts der mehreren Öffnungen (4) in der inneren Wandung (40) des Schlauchs (1).

14. Verfahren nach Anspruch 12 oder 13, **dadurch gekennzeichnet, dass** vor Schritt A) der Schlauch (1) durch Abschneiden gekürzt wird und ein Verschlusselement (5) in das gerade geschnittene Ende des Schlauchs (1) gesteckt oder geschraubt wird, wobei das Verschlusselement (5) den Schlauch (1) fluiddicht abschließt, bevorzugt fluiddicht und druckdicht verschließt.

15. Verfahren nach einem der Ansprüche 12 bis 14, **dadurch gekennzeichnet, dass** das Verfahren mit einer Vorrichtung nach einem der Ansprüche 1 bis 11 durchgeführt wird.

## Claims

1. A device for local administration of a medical fluid, having
a hose (1), which is flexibly deformable and which has a hose wall,
wherein the hose wall has an outer wall (38) of a first material which is arranged radially to the outside, and the hose wall has an inner wall (40) of a second material which is arranged radially to the inside and which delimits an inner conduit (30) of the hose (1), wherein, in a distal portion (2) of the hose (1), the hose (1) has multiple orifices (4) in the hose wall, wherein the multiple orifices (4) connect the inner conduit (30) of the hose (1) with the surroundings of the hose (1), wherein the distal portion (2) of the hose (1) is delimited by a distal end of the hose (1), wherein
the multiple orifices (4) in the inner wall (40) are reversibly closable in fluid-tight manner as a function of a physical quantity acting on the second material, in particular as a function of a pressure from the medical fluid acting on the second material, or
the multiple orifices (4) in the outer wall (38) are reversibly closable in fluid-tight manner as a function of a physical quantity acting on the first material, in particular as a function of a pressure from the medical fluid acting on the first material,
wherein, in a proximal portion (3) of the hose (1), the hose (1) does not have any orifices (4) in the hose wall which connect the inner conduit (30) of the hose (1) with the surroundings of the hose (1) in a liquid-permeable manner,
the device further having a closing element (5) with which the hose (1) is closed or closable in liquid-tight manner at the distal end of the hose (1), wherein the closing element (5) is manually insertable into the distal end of the hose (1),
wherein a proximal end of the hose (1) is connected or connectable to a container (7) for the medical fluid, in a liquid-permeable manner, in such a way that the medical fluid is pressable out of the container (7) through the proximal end of the hose (1) into the inner conduit (30) of the hose (1) and pressed out to the surroundings of the hose (1) through the multiple orifices (4).

2. The device according to Claim 1, **characterized in that**
the inner conduit (30) of the hose (1) begins at a proximal opening in the proximal end of the hose (1) and ends at a distal opening in the distal end of the hose (1), wherein the distal opening of the hose (1) is closed or closable by the closing element (5), and/or the device comprises the container (7) for the medical fluid, wherein the container (7) preferably comprises a hollow cylinder with a plunger (8) displaceable axially in the hollow cylinder, said plunger closing a first end of the hollow cylinder, wherein the hollow cylinder has a discharge opening at an opposite end from the first end, said discharge opening being connected or connectable with the proximal end of the hose (1), preferably being connected or connectable with the proximal end of the hose (1) via a manually operable valve element (9) for regulating the flow velocity of the medical fluid, wherein preferably
a medical fluid, in particular a pharmaceutical fluid, is contained in the container (7).

3. The device according to any one of the preceding claims, **characterized in that** the device has a delivery means (6), with which the medical fluid is pressable out of the connected or connectable container (7) into the hose (1), through the inner conduit (30) of the hose (1) and through the multiple orifices (4) into the surroundings of the hose (1), wherein preferably
the delivery means (6) has an energy storage element, in particular at least one tensioned spring (18), wherein the delivery means (6) is drivable with energy from the energy storage element, wherein particular preferably a plunger (8) is drivable with the energy storage element in a hollow cylinder as the container (7) towards an opposing discharge opening.

4. The device according to any one of the preceding claims, **characterized in that** the outer wall (38) and the inner wall (40) are fixedly connected to each other, preferably connected to each other over the entire surface, and
the sum of the free cross-sectional areas of all the multiple orifices (4) are at most as large as the free cross-section of the inner conduit (30).

5. The device according to any one of the preceding claims, **characterized in that** the multiple orifices (4) in the outer wall (38) of the hose wall are open irrespective of the pressure applied by the medical fluid, while the multiple orifices (4) in the inner wall (40) of the hose wall are closed when no pressure is applied by the medical fluid and are openable in a fluid-permeable manner by applying pressure on the medical fluid, and/or
the outer wall (38) of the hose wall absorbs a pressure from the medical fluid in the inner conduit (30) imparted via the inner wall (40) of the hose wall, without expanding radially by more than 5%, preferably without expanding radially by more than 1%, or the hose (1) expands radially by at most 5 per cent under an internal pressure of 500 kPa relative to normal pressure, preferably by at most 1 per cent under an internal pressure of 500 kPa relative to normal pressure.

6. The device according to any one of the preceding claims, **characterized in that** the closing element (5) has a conical or cylindrical projection which is inserted or screwed into the hose (1), such that the hose (1) is clamped in the region of the distal end of the outer hose (1) by the projection in such a way that the hose (1) is closed in fluid-tight manner at the distal end of the hose (1), wherein the conical or cylindrical projection preferably has ribs on the outside of the projection or the conical or cylindrical projection has an external thread (28), wherein the external thread (28) or the conical or cylindrical projection has a larger external diameter then the internal diameter of the hose (1).

7. The device according to any one of the preceding claims, **characterized in that** the multiple orifices (4) in the distal portion (2) of the hose (1) in the outer wall (38) have a diameter of at most 500 µm, preferably of at most 250 µm and particularly preferably of at most 100 µm, and/or
the hose (1) is formed of a coaxial coextrudate, wherein the inner wall (40) consists of a rubber-elastic polymer, in particular polyurethane or a weakly crosslinked polymer, and the outer wall (38) consists of a non-rubber-elastic thermoplastic polymer or of a highly crosslinked polymer, in particular of polyamide.

8. The device according to any one of the preceding claims, **characterized in that** an X-ray-opaque material is present in the hose (1) at least at the distal end of the hose (1) and/or in the closing element (5), preferably an X-ray-opaque material is present in the distal portion (2) of the hose (1) and/or in the closing element (5), particularly preferably is present over the entire length of the distal portion (2) of the hose (1) and in the closing element (5) or is present over the entire length of the hose (1) and in the closing element (5), and/or
at least one metal wire (32), at least one metal coil and/or at least one metal mesh is/are arranged in the inner conduit (30) of the hose (1) and/or in the hose wall of the hose (1), wherein the at least one metal wire (32), the at least one metal coil and/or the at least one metal mesh are preferably arranged along the entire length of the hose (1).

9. The device according to any one of the preceding claims, **characterized in that** the closing element (5) has the following features:
a rotationally symmetrical first body with an external thread (28) or with circumferentially extending ribs, wherein the external thread (28) has or the ribs have a larger external diameter than the internal diameter of the hose (1),
a rotationally symmetrical second body with an external diameter less than or equal to the external diameter of the hose (1), wherein the axial extent of the second body is at least 5 mm,
wherein the rotationally symmetrical first body is axially connected with the rotationally symmetrical second body, and/or
the closing element (5) is screwed or pressed into the distal end of the hose (1) and closes the free line cross-section of the inner conduit (30) of the hose (1) at the distal end completely and in liquid-tight and pressure-tight manner.

10. The device according to any one of the preceding claims, **characterized in that** the hose (1) is plastically deformable and has an external diameter of less than or equal to 7 mm, preferably an external diameter of between 2 mm and 4 mm, and
the first material has a greater Shore A hardness than the second material, wherein the first material preferably has a Shore A hardness of more than 60 and the second material a Shore A hardness of less than 60, and/or
the multiple orifices (4) in the inner wall (40) have a free cross-section greater by a factor of two or more when pressure with a hydrostatic pressure of 500 kPa is applied thereto by the medical fluid compared to without applying pressure, wherein preferably the multiple orifices (4) in the inner wall (40) or the outer wall (38) are slit-shaped.

11. The device according to any one of the preceding claims, **characterized in that** the multiple orifices (4) are reversibly openable by elastic deformation of the second material, while the multiple orifices (4) in the first material remain open, wherein the first material is preferably dimensionally stable such that the outer wall (38) absorbs at least some of the forces caused by the elastic deformation of the second material and thereby counteracts radial deformation of the hose (1).

12. A method for operating a medical device for local administration of a medical fluid, wherein the medical fluid is not delivered to a human or animal body in the context of the method, the device having a hose (1) with a hose wall, wherein the hose wall has an outer wall (38) of a first material which is arranged radially to the outside, and the hose wall has an inner wall (40) of a second material which is arranged radially to the inside and which delimits an inner conduit (30) of the hose (1), wherein the hose (1) has multiple orifices (4) in the hose wall, wherein the multiple orifices (4) connect the inner conduit (30) of the hose (1) with the surroundings of the hose (1), the method having the following steps:
A) introducing a medical fluid into the hose (1);
B) exerting pressure on the medical fluid in the hose (1);
C) opening the multiple orifices (4) in the inner wall (40) or in the outer wall (38) of the hose (1) through the pressure of the medical fluid acting on the multiple orifices (4); and
D) expelling medical fluid through the open multiple orifices (4).

13. The method according to Claim 12, **characterized in that** step E) reducing the pressure on the medical fluid in the hose (1) after step D) and thereby closing the multiple orifices (4) in the inner wall (40) or in the outer wall (38) of the hose (1) or reducing the free cross-section of the multiple orifices (4) in the inner wall (40) or in the outer wall (38) of the hose (1), and/or that

14. The method according to Claim 12 or 13, **characterized in that** the hose (1) is shortened by cutting off prior to step A) and a closing element (5) is inserted or screwed into the end of the hose (1) which has just been cut, wherein the closing element (5) seals the hose (1) in fluid-tight manner, preferably closes it in fluid-tight and pressure-tight manner.

15. The method according to any one of Claims 12 to 14, **characterized in that** the method is carried out using a device according to any one of Claims 1 to 11.

## Revendications

1. Dispositif pour l'application locale d'un fluide médical présentant
un tuyau (1), lequel est déformable de manière flexible et présente une paroi de tuyau,
la paroi de tuyau présentant une paroi extérieure (38) constituée d'un premier matériau, laquelle est disposée radialement à l'extérieur, et la paroi de tuyau présentant une paroi intérieure (40) constituée d'un second matériau, laquelle est disposée radialement à l'intérieur et laquelle limite une conduite intérieure (30) du tuyau (1),
le tuyau (1) présentant dans une partie distale (2) du tuyau (1) une pluralité d'ouvertures (4) dans la paroi de tuyau, la pluralité d'ouvertures (4) connectant la conduite intérieure (30) du tuyau (1) avec l'environnement du tuyau (1),
la partie distale (2) du tuyau (1) étant limitée par une extrémité distale du tuyau (1),
la pluralité d'ouvertures (4) dans la paroi intérieure (40) pouvant, en fonction d'une grandeur physique agissant sur le second matériau, en particulier en fonction d'une pression agissant sur le second matériau à partir du fluide médical, être fermée de manière étanche aux fluides ou
la pluralité d'ouvertures (4) dans la paroi extérieure (38) pouvant, en fonction d'une grandeur physique agissant sur le premier matériau, en particulier en fonction d'une pression agissant sur le premier matériau à partir du fluide médical, être fermées de manière étanche aux fluides,
le tuyau (1) ne présentant dans une partie proximale (3) du tuyau (1) aucune ouverture (4) dans la paroi de tuyau, qui connectent de manière perméable aux liquides la conduite intérieure (30) du tuyau (1) avec l'environnement du tuyau (1),
le dispositif présentant en outre un élément de fermeture (5), avec lequel le tuyau (1) est ou peut être fermé de manière étanche aux liquides à l'extrémité distale du tuyau (1), l'élément de fermeture (5) pouvant être monté manuellement dans l'extrémité distale du tuyau (1), une extrémité proximale du tuyau (1) étant ou pouvant être connectée de manière perméable aux liquides avec un réservoir (7) pour le fluide médical de telle façon que le fluide médical puisse être pressé du réservoir (7) à travers l'extrémité proximale du tuyau (1) dans la conduite intérieure (30) du tuyau (1) et puisse être extrait à travers la pluralité d'ouvertures (4) dans l'environnement du tuyau (1).

2. Dispositif selon la revendication 1, **caractérisé en ce que**
la conduite intérieure (30) du tuyau (1) commence au niveau d'une ouverture proximale à l'extrémité proximale du tuyau (1) et se termine au niveau d'une ouverture distale à l'extrémité distale du tuyau (1), l'ouverture distale du tuyau (1) étant ou pouvant être fermée par l'élément de fermeture (5), et/ou
le dispositif présente le réservoir (7) pour le fluide médical, le réservoir (7) présentant de préférence un cylindre creux comprenant un piston (8) déplaçable axialement dans le cylindre creux, lequel ferme une première extrémité du cylindre creux, le cylindre creux présentant à une extrémité opposée à la première extrémité une ouverture de sortie, laquelle est ou peut être connectée à l'extrémité proximale du tuyau (1), étant ou pouvant être de préférence connectée à l'extrémité proximale du tuyau (1) à travers un élément de soupape (9) pouvant être actionné manuellement pour le réglage de la vitesse d'écoulement du fluide médical, préférentiellement
un fluide médical, en particulier un fluide pharmaceutique, étant contenu dans le réservoir (7).

3. Dispositif selon l'une quelconque des revendications précédentes, **caractérisé en ce que** le dispositif présente un dispositif de transport (6), au moyen duquel le fluide médical est pressé hors du réservoir (7) connecté ou pouvant être connecté dans le tuyau (1), à travers la conduite intérieure (30) du tuyau (1) et à travers la pluralité d'ouvertures (4) dans l'environnement du tuyau (1), préférentiellement
le dispositif de transport (6) présentant un élément accumulateur d'énergie, en particulier un ressort (18) tendu, le dispositif de transport (6) pouvant être entraîné au moyen d'énergie provenant de l'élément accumulateur d'énergie, de manière particulièrement préférée un piston (8) étant entraîné dans un cylindre creux comme réservoir (7) dans la direction d'une ouverture de sortie opposée au moyen de l'élément accumulateur d'énergie.

4. Dispositif selon l'une quelconque des revendications précédentes, **caractérisé en ce que** la paroi extérieure (38) et la paroi intérieure (40) sont connectées l'une à l'autre de manière fixe, sont de préférence connectées l'une à l'autre sur toute leur surface, et
la somme des superficies de sections transversales de toutes les ouvertures de la pluralité d'ouvertures (4) est au maximum aussi grande que la section transversale libre de la conduite intérieure (30).

5. Dispositif selon l'une quelconque des revendications précédentes, **caractérisé en ce que**
la pluralité d'ouvertures (4) dans la paroi extérieure (38) de la paroi de tuyau est ouverte indépendamment de la pression du fluide médical, tandis que la pluralité d'ouvertures (4) dans la paroi intérieure (40) de la paroi de tuyau est fermée sans sollicitation par pression par le fluide médical et est ouverte de manière perméable aux liquides par une pression sur le fluide médical et/ou
la paroi extérieure (38) de la paroi de tuyau reçoit dans la conduite intérieure (30) une pression du fluide médical transmise à travers la paroi intérieure (40) de la paroi de tuyau, sans se dilater radialement de plus de 5 %, de préférence sans se dilater radialement de plus de 1 %, ou
le tuyau (1) se dilate radialement, pour une pression intérieure de 500 kPa par rapport à la pression normale, de maximum 5 pour cent, de préférence de maximum 1 pour cent.

6. Dispositif selon l'une quelconque des revendications précédentes, **caractérisé en ce que** l'élément de fermeture (5) présente une saillie conique ou cylindrique, laquelle est insérée ou vissée dans le tuyau (1), de sorte que le tuyau (1), dans la zone de l'extrémité distale du tuyau (1) extérieur, est tendu par la saillie de telle façon que le tuyau (1) est fermé de manière étanche aux fluides au niveau de l'extrémité distale du tuyau (1), la saillie conique ou cylindrique présentant préférentiellement des traverses sur le côté extérieur de la saillie ou la saillie conique ou cylindrique présentant un filetage extérieur (28), le filetage extérieur (28) ou la saille conique ou cylindrique présentant un diamètre supérieur au diamètre intérieur du tuyau (1).

7. Dispositif selon l'une quelconque des revendications précédentes, **caractérisé en ce que** la pluralité d'ouvertures (4) dans la partie distale (2) du tuyau (1) dans la paroi extérieure (38) a un diamètre d'au maximum 500 µm, de préférence d'au maximum 250 µm et de manière particulièrement préférée d'au maximum 100 µm, et/ou le tuyau (1) est formé par un co-extrudat coaxial, la paroi intérieure (40) étant constituée d'un polymère caoutchouteux, en particulier de polyuréthane, ou d'un polymère faiblement réticulé et la paroi extérieure (38) étant constituée d'un polymère thermoplastique non caoutchouteux ou d'un polymère fortement réticulé, en particulier de polyamide.

8. Dispositif selon l'une quelconque des revendications précédentes, **caractérisé en ce que** un matériau radio-opaque est contenu dans le tuyau (1) au moins au niveau de l'extrémité distale du tuyau (1) et/ou dans l'élément de fermeture (5), un matériau radio-opaque est de préférence contenu dans la partie distale (2) du tuyau (1) et/ou dans l'élément de fermeture (5), est de manière particulièrement préférée contenu sur toute la longueur de la partie distale (2) du tuyau (1) et dans l'élément de fermeture (5) ou sur toute la longueur du tuyau (1) et dans l'élément de fermeture (5), et/ou au moins un fil métallique (32), au moins une spirale métallique et/ou au moins un filet métallique est ou sont disposé(s) dans la conduite intérieure (30) du tuyau (1) et/ou dans la paroi de tuyau du tuyau (1), l'au moins un fil métallique (32), l'au moins une spirale métallique et/ou l'au moins un filet métallique étant disposés de préférence le long de toute la longueur du tuyau (1).

9. Dispositif selon l'une quelconque des revendications précédentes, **caractérisé en ce que** l'élément de fermeture (5) présente les caractéristiques suivantes :
un premier corps symétrique en rotation comprenant un filetage extérieur (28) ou comprenant des traverses réparties sur la circonférence, le filetage extérieur (28) ou les traverses possédant un diamètre extérieur supérieur au diamètre intérieur du tuyau (1), un second corps symétrique en rotation comprenant un diamètre extérieur inférieur ou égal au diamètre extérieur du tuyau (1), l'extension axiale du second corps étant d'au moins 5 mm, le premier corps symétrique en rotation étant connecté axialement avec le second corps symétrique en rotation, et/ou
l'élément de fermeture (5) est vissé ou enfoncé dans l'extrémité distale du tuyau (1) et ferme de manière complètement étanche aux liquides et étanche à la pression la section transversale de conduite libre de la conduite intérieure (30) du tuyau (1) à l'extrémité distale.

10. Dispositif selon l'une quelconque des revendications précédentes, **caractérisé en ce que** le tuyau (1) est déformable plastiquement et présente un diamètre extérieur inférieur ou égal à 7 mm, de préférence un diamètre extérieur compris entre 2 mm et 4 mm, et
le premier matériau a une dureté Shore A supérieure à celle du second matériau, le premier matériau ayant de préférence une dureté Shore A supérieure à 60 et le second matériau une dureté Shore A inférieure à 60, et/ou
la pluralité d'ouvertures (4) dans la paroi intérieure (40) présente, lors de la sollicitation par pression avec une pression hydrostatique de 500 kPa par le fluide médical, une section transversale libre supérieure d'un facteur deux ou plus à la section transversale libre sans sollicitation par pression, préférentiellement
la pluralité d'ouvertures (4) dans la paroi intérieure (40) ou dans la paroi extérieure (38) est formée en forme de fente.

11. Dispositif selon l'une quelconque des revendications précédentes, **caractérisé en ce que** la pluralité d'ouvertures (4) est ouverte de manière réversible par une déformation élastique du second matériau, tandis que la pluralité d'ouvertures (4) dans le premier matériau reste ouverte, le premier matériau étant indéformable de telle façon que la paroi extérieure (38) reçoit au moins une partie des forces engendrées par la déformation élastique du second matériau et contrecarre ainsi une déformation radiale du tuyau (1).

12. Procédé pour faire fonctionner un dispositif médical pour l'application locale d'un fluide médical, le fluide médical dans le cadre du procédé n'étant pas fourni sur un corps humain ou animal, le dispositif présentant un tuyau (1) comprenant une paroi de tuyau, la paroi de tuyau présentant une paroi extérieure (38) constituée d'un premier matériau, laquelle est disposée radialement à l'extérieur, et la paroi de tuyau présentant une paroi intérieure (40) constituée d'un second matériau, laquelle est disposée radialement à l'intérieur et laquelle limite une conduite intérieure (30) du tuyau (1), le tuyau (1) présentant une pluralité d'ouvertures (4) dans la paroi de tuyau, la pluralité d'ouvertures (4) connectant la conduite intérieure (30) du tuyau (1) avec l'environnement du tuyau (1), le procédé présentant les étapes suivantes :
A) introduction d'un fluide médical dans le tuyau (1) ;
B) application d'une pression sur le fluide médical dans le tuyau (1) ;
C) ouverture de la pluralité d'ouvertures (4) dans la paroi intérieure (40) ou dans la paroi extérieure (38) du tuyau (1) au moyen de la pression du fluide médical agissant sur la pluralité d'ouvertures (4) ; et
D) expulsion du fluide médical à travers la pluralité d'ouvertures (4) ouvertes.

13. Procédé selon la revendication 12, **caractérisé par**
l'étape E) réduction de la pression sur le fluide médical dans le tuyau (1) après l'étape D) et par la fermeture de la pluralité d'ouvertures (4) dans la paroi intérieure (40) du tuyau (1) ou diminution de la section transversale libre de la pluralité d'ouvertures (4) dans la paroi intérieure (40) du tuyau (1).

14. Procédé selon la revendication 12 ou 13, **caractérisé en ce que**,
avant l'étape A), le tuyau (1) est raccourci par découpe et un élément de fermeture (5) est inséré ou vissé dans l'extrémité tout juste coupée du tuyau (1), l'élément de fermeture (5) fermant le tuyau (1) de manière étanche aux fluides, de préférence étanche aux fluides et étanche à la pression.

15. Procédé selon l'une quelconque des revendications 12 à 14, **caractérisé en ce que** le procédé est exécuté avec un dispositif selon l'une quelconque des revendications 1 à 11.
